# EUROPEAN PATENT APPLICATION

(11) **EP 4 477 655 A1**
(43) Date of publication of application: **18.12.2024**
(21) Application number: 22924943.8
(22) Date of filing: 04.11.2022
(51) Int. Cl.: C07D 498/04, A61K 31/5383, A61K 31/7115, A61K 48/00, A61P 21/00, C07H 19/23, C12N 15/113, C12N 15/115

(54) **PHENOXAZINE DERIVATIVE AND DRUG DELIVERY SYSTEM FORMULATION USING SAME**

(30) Priority: 07.02.2022 JP 2022017564
(71) Applicant: OSAKA UNIVERSITY, Suita-shi Osaka 565-0871 (JP)
(72) Inventor: OBIKA, Satoshi, Suita-shi, Osaka 565-0871 (JP); NAKAGAWA, Osamu, Suita-shi, Osaka 565-0871 (JP); YAMAJI, Ryohei, Suita-shi, Osaka 565-0871 (JP); KAMADA, Haruhiko, Ibaraki-shi, Osaka 567-0085 (JP); NAKAYAMA, Taisuke, Ibaraki-shi, Osaka 567-0085 (JP)
(74) Representative: Schlich
(86) International application number: PCT/JP2022/041129
(87) International publication number: WO 2023/149038

(57) **Abstract**

A compound or a pharmaceutically acceptable salt thereof according to the present invention has a structure represented by Formula (I) and can form a ligand for a drug delivery system formulation. The drug delivery system formulation according to the present invention can improve, for example, an ability to make a delivery to the skeletal muscle.

## Description

### Technical Field

The present invention relates to a phenoxazine derivative and a drug delivery system formulation containing the same.

### Background Art

An antisense drug, which is one type of nucleic acid drug, controls the functions or expression of a disease-causing protein by forming a double strand of a target mRNA and a complementary antisense oligonucleotide (ASO). The antisense drug can target a gene such as mRNA or miRNA, and if the sequence is known, a drug can be quickly developed. Therefore, the antisense drug is expected as a novel drug development modality with potential for new treatment of an intractable disease.

On the other hand, a nucleic acid drug has a problem in that it has poor cell membrane permeability and is easily excreted through the kidneys. As a strategy to address this problem, a receptor-selective ligand is incorporated in an oligonucleotide. For example, binding of the ligand to a receptor expressed on the surface of a cell membrane makes it possible to induce endocytosis and effectively deliver the oligonucleotide into a cell.

A representative example of the application of this strategy is an oligonucleotide to which N-acetylgalactosamine (GalNAc) is conjugated (Non-Patent Document 1). Non-Patent Document 1 states that conjugation of GalNac to a terminus of an oligonucleotide leads to induction of endocytosis via an asialoglycoprotein receptor expressed on the surface of the cell membrane of a hepatic mesenchymal cell in the liver and to success in efficient accumulation of the oligonucleotide in the liver.

For example, givosiran was approved as a world's first ligand-conjugated siRNA in 2019 and lumasiran and inclisiran, which are also ligand-conjugated siRNAs, were approved in 2020, and therefore, the strategy of conjugating a ligand to an oligonucleotide is very promising.

Currently, there is increasing demand for delivery of nucleic acid drugs to viscera other than the liver, and an oligonucleotide to which a glucagon-like peptide-1 (eGLP-1) for delivery to the pancreas is conjugated (Non-Patent Document 2), palmitic acid-conjugated oligonucleotide having an effect on delivery to the muscular tissue (Non-Patent Document 3), and the like have been reported. However, none of these have been put to practical use, and it is still difficult to deliver nucleic acid drugs to viscera other than the liver.

### Related Art Document

### Non-Patent Documents

Non-Patent Document 1: T P. Prakash et al., Nucleic Acid Res., 42, 8796-8807 (2014).
Non-Patent Document 2: C. Ammala et al., Sci. Adv., 4 (2018) DOI: 10.1126/sciadv.aat3386.
Non-Patent Document 3: A. E. Chappellet al., Nucleic Acids Res., 48, 4382-4395 (2020).

### Summary of Invention

### Problem to be Solved by the Invention

The present invention is to solve the above-mentioned problems, and it is an object thereof to provide a compound that enables delivery of a nucleic acid drug to a viscus, an organ, or a tissue other than the liver, and a drug delivery system formulation containing the compound.

### Means for Solving the Problem

The present invention is a compound represented by Formula (I) below or a pharmaceutically acceptable salt thereof 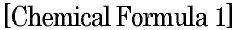 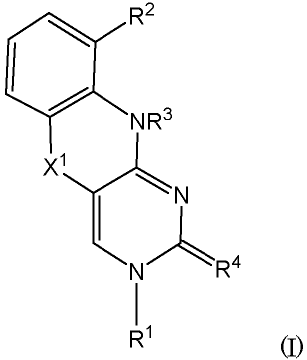 (in Formula (I),
R¹ is a group represented by Formula (II) below: 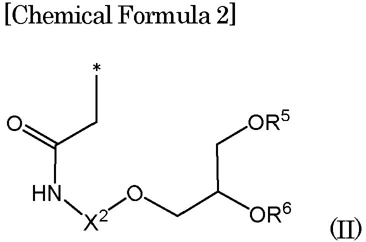 (in Formula (II),
   X² is a group represented by the formula below: 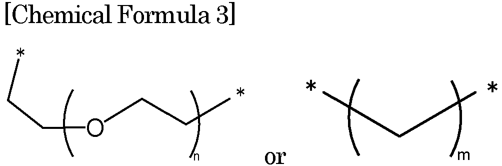 (where n is any of integers of 2 to 4,
      m is any of integers of 2 to 4, and
      * is an atomic bonding),
   R⁵ is a hydrogen atom, an alkyl group having 1 to 7 carbon atoms that may be branched or cyclic, or a hydroxy group protecting group for nucleic acid synthesis,
   R⁶ represents a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from an α group and may include a heteroatom, an aralkyl group with an aryl moiety having 3 to 12 carbon atoms that may have any one or more substituents selected from the α group and may include a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or P(R¹⁴)R¹⁵ (where R¹⁴ and R¹⁵ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms),
      the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms, and
   * is an atomic bonding),
R² is a hydrogen atom, a hydroxy group, a nitro group, an amino group, or a group represented by Formula (III) below: 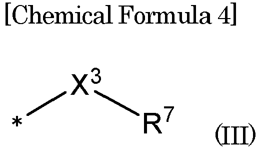 (in Formula (III),
   X³ is an oxygen atom or a sulfur atom, and
   R⁷ is a group represented by the formula below: 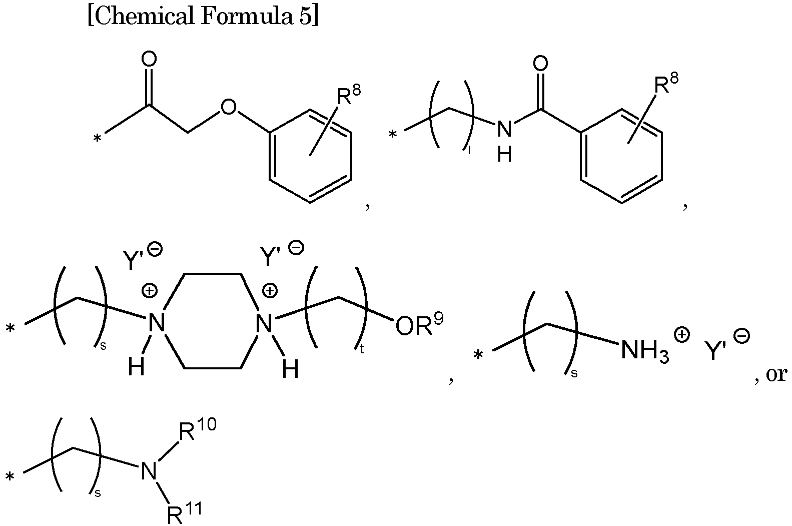 (where
      R⁸ is a hydrogen atom, a hydroxy group, an alkyl group having 1 to 7 carbon atoms that may be branched or cyclic, or an alkoxy group having 1 to 7 carbon atoms that may be branched or cyclic,
      R⁹ is a hydrogen atom, or an alkyl group having 1 to 7 carbon atoms that may be branched or cyclic,
      R¹⁰ and R¹¹ are each independently a hydrogen atom, an alkyl group having 1 to 7 carbon atoms that may be branched or cyclic, or an amino group protecting group,
      Y' is a pharmaceutically acceptable anion,
      s is any of integers of 2 to 4,
      t is any of integers of 2 to 4,
      l is any of integers of 1 to 5, and
      * is an atomic bonding)),
R³ is a hydrogen atom, an alkyl group having 1 to 7 carbon atoms that may be branched or cyclic, or an amino group protecting group,
R⁴ is an oxygen atom or a sulfur atom, and
X¹ is an oxygen atom or a sulfur atom).

In one embodiment, Formula (I) is represented by Formula (Ia) below: 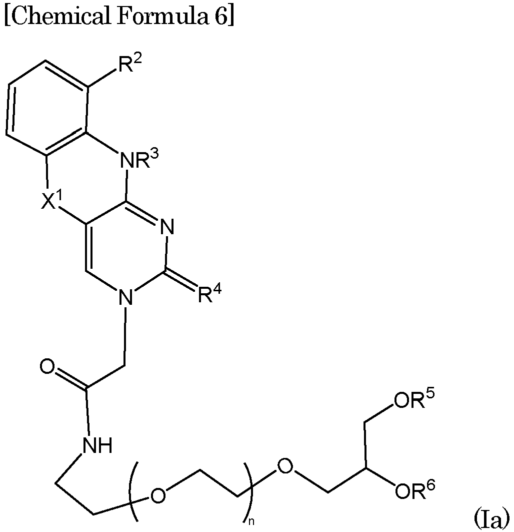 (in Formula (Ia), R², R³, R⁴, R⁵, R⁶, X¹, and n are as defined above).

In one embodiment, Formula (I) is represented by Formula (Ib) below: 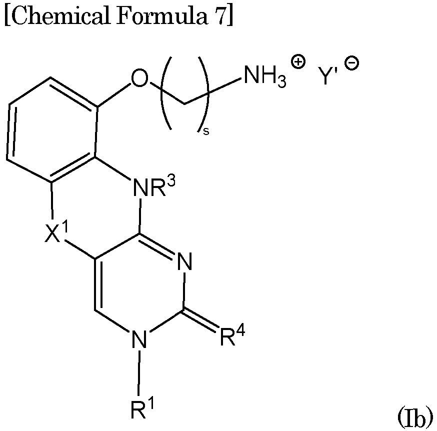 (in Formula (Ib), R¹, R³, R⁴, X¹, Y', and s are as defined above).

In one embodiment, Formula (I) is represented by Formula (Ic) below: 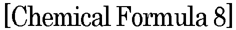 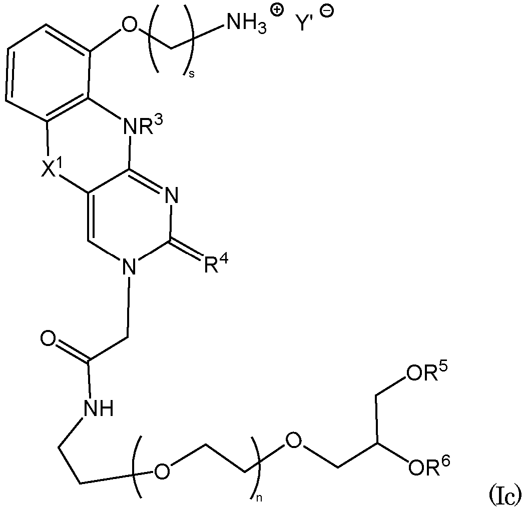 (in Formula (Ic), R³, R⁴, R⁵, R⁶, X¹, Y', n, and s are as defined above).

The present invention is also a drug delivery system formulation comprising a ligand derived from the above compound or pharmaceutically acceptable salt thereof.

The present invention is also a drug delivery system formulation comprising
a ligand derived from a compound represented by Formula (I') below or a pharmaceutically acceptable salt thereof 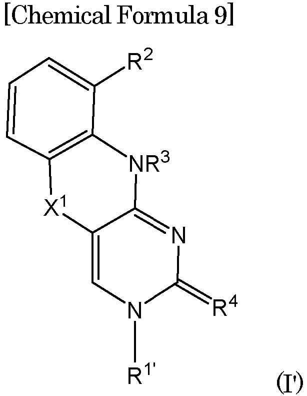 (in Formula (I'),
   R^{1'}is
   a hydrogen atom, an alkyl group having 1 to 7 carbon atoms that may be branched or cyclic, an alkoxy group having 1 to 7 carbon atoms that may be branched or cyclic, or an alkylthio group having 1 to 6 carbon atoms that may be branched; or
   a pentose moiety that includes a chemical structure selected from the group consisting of a ribos- 1-yl structure and a 2-deoxyribos-1-yl structure,
   R² is a hydrogen atom, a hydroxy group, a nitro group, an amino group, or a group represented by Formula (III) below: 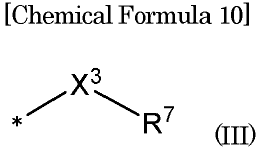 (in Formula (III),
      X³ is an oxygen atom or a sulfur atom, and
R⁷ is a group represented by the formula below: 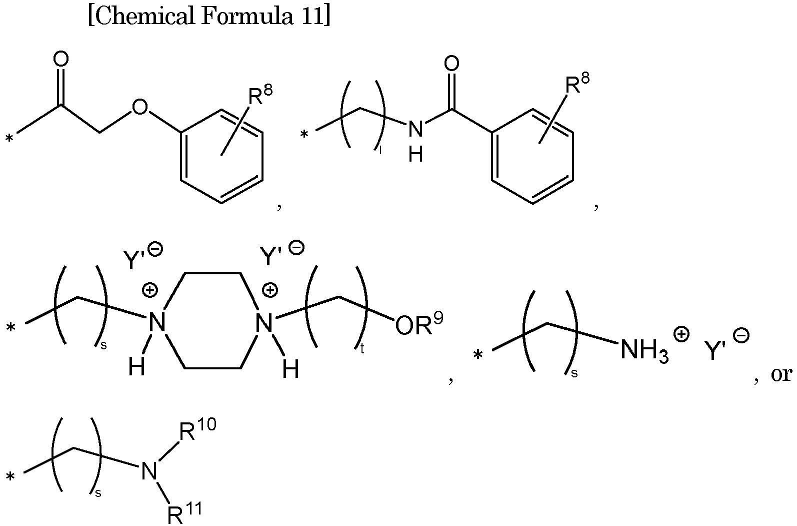 (where
   R⁸ is a hydrogen atom, a hydroxy group, an alkyl group having 1 to 7 carbon atoms that may be branched or cyclic, or an alkoxy group having 1 to 7 carbon atoms that may be branched or cyclic,
   R⁹ is a hydrogen atom, or an alkyl group having 1 to 7 carbon atoms that may be branched or cyclic,
   R¹⁰ and R¹¹ are each independently a hydrogen atom, an alkyl group having 1 to 7 carbon atoms that may be branched or cyclic, or an amino group protecting group,
   Y' is a pharmaceutically acceptable anion,
   s is any of integers of 2 to 4,
   t is any of integers of 2 to 4,
   l is any of integers of 1 to 5, and
   * is an atomic bonding)),
R³ is a hydrogen atom, an alkyl group having 1 to 7 carbon atoms that may be branched or cyclic, or an amino group protecting group,
R⁴ is an oxygen atom or a sulfur atom, and
X¹ is an oxygen atom or a sulfur atom).

In one embodiment, R^{1'} in Formula (I') is the pentose moiety.

In a further embodiment, the pentose moiety is a moiety obtained through desorption of a base moiety from an artificial nucleic acid monomer.

In a more further embodiment, the artificial nucleic acid monomer is a 2',4'-BNA/LNA monomer.

In one embodiment, the ligand is contained in the form of a conjugate where the ligand is linked to a polynucleic acid.

In a further embodiment, the ligand is linked to the 5' terminus of the polynucleic acid.

In a further embodiment, in the conjugate, the number of the ligands linked to the polynucleic acid is one to three per polynucleic acid molecule.

In a further embodiment, the polynucleic acid is an oligonucleotide.

In a more further embodiment, the oligonucleotide is an antisense oligonucleotide, siRNA, or nucleic acid aptamer.

In one embodiment, the drug delivery system formulation of the preset invention is used for delivery to a skeletal muscle.

The present invention is also a pharmaceutical comprising the above drug delivery system formulation.

### Effects of the Invention

With the present invention, it is possible to deliver a nucleic acid drug to a viscus, an organ, or a tissue other than the liver, such as the skeletal muscle. The drug delivery system formulation according to the present invention is safe for a living organism and can be efficiently produced without need for a complicated production process.

### Brief Description of Drawings

FIG. 1 is a graph showing a fluorescence spectrum of a single stranded oligonucleotide (ODN 1X) produced in Example 2 in which a single G-clamp linker moiety is introduced to the 5' terminus, and fluorescence spectra of double strands formed by the oligonucleotide and complementary DNA.
FIG. 2 is a graph showing a fluorescence spectrum of a single stranded oligonucleotide (ODN 2X) produced in Example 2 in which three G-clamp linker moieties are introduced to the 5' terminus, and fluorescence spectra of double strands formed by the oligonucleotide and complementary DNA.
FIG. 3 is a graph showing a fluorescence spectrum of a single stranded oligonucleotide (ODN 1X) produced in Example 2 in which a single G-clamp linker moiety is introduced to the 5' terminus, and fluorescence spectra of double strands formed by the oligonucleotide and complementary RNA.
FIG. 4 is a graph showing a fluorescence spectrum of a single stranded oligonucleotide (ODN 2X) produced in Example 2 in which three G-clamp linker moieties are introduced to the 5' terminus, and fluorescence spectra of double strands formed by the oligonucleotide and complementary RNA.
FIG. 5 shows graphs showing expression levels of mMALAT1 RNA in the skeletal muscles of mice after administration of nucleic acid produced in Example 4 to the mice. The data in the diagrams are shown as "average value ± standard deviation" (n=5). In FIG. 5, * indicates P<0.05 and ** indicates P<0.01, indicating significant differences with respect to the control group (saline). In FIG. 5, horizontal lines with a P value indicate significant differences between groups. FIG. 5c collectively shows the results shown in FIGS. 5a and 5b.

### Description of Embodiments

### Definitions of Terms

The terms as used herein are used in the general senses in the art unless otherwise stated.

First, typical terms as used herein are defined.

The term "alkyl group having 1 to n carbon atoms" as used herein refers to any linear, branched, or cyclic alkyl group having 1 to n carbon atoms. For example, when the number of carbon atoms n is 9, examples of the alkyl group having 1 to n carbon atoms include a methyl group, an ethyl group, an n-propyl group, an isopropyl group, an n-butyl group, an s-butyl group, a t-butyl group, an n-pentyl group, an isopentyl group, a neopentyl group, a t-pentyl group, an n-hexyl group, an isohexyl group, an n-heptyl group, an n-octyl group, and an n-nonyl group. In addition, for example, the term "alkyl group having 1 to 9 carbon atoms that may be branched" refers to a linear or branched alkyl group having 1 to 9 carbon atoms. The term "alkyl group having 1 to 3 carbon atoms that may be branched" refers to any linear or branched alkyl group having 1 to 3 carbon atoms. Furthermore, the term "linear alkyl group having 1 to 6 carbon atoms" refers to any linear alkyl group having 1 to 6 carbon atoms. Also, the term "alkyl group having 1 to 7 carbon atoms that may be branched or cyclic" refers to any linear, branched, or cyclic alkyl group having 1 to 7 carbon atoms.

The term "alkoxy group having 1 to n carbon atoms" as used herein refers to any linear, branched, or cyclic alkoxy group having 1 to n carbon atoms. For example, when the number of carbon atoms n is 7, examples of the alkoxy group having 1 to n carbon atoms include a methoxy group, an ethoxy group, an n-propoxy group, an isopropoxy group, an n-butoxy group, an s-butoxy group, a t-butoxy group, an n-pentoxy group, an isopentoxy group, a neopentoxy group, an n-hexoxy group, an isohexoxy group, a t-hexoxy group, a neohexoxy group, a cyclohexoxy group, an n-heptoxy group, an isoheptoxy group, a neoheptoxy group, and t-heptoxy group. In addition, for example, the term "alkoxy group having 1 to 3 carbon atoms that may be branched" refers to a linear or branched alkoxy group having 1 to 3 carbon atoms. The term "linear alkoxy group having 1 to 6 carbon atoms" encompasses alkoxy groups that include any linear alkyl group having 1 to 6 carbon atoms. Meanwhile, the term "alkoxy group having 1 to 6 carbon atoms" refers to any linear, branched, or cyclic alkoxy group having 1 to 6 carbon atoms. Also, the term "alkoxy group having 1 to 7 carbon atoms that may be branched or cyclic" refers to any linear, branched, or cyclic alkoxy group having 1 to 7 carbon atoms.

The term "linear alkoxy group having 1 to 6 carbon atoms that may have undergone substitution by a linear alkoxy group having 1 to 6 carbon atoms" refers to the above-mentioned "linear alkoxy group having 1 to 6 carbon atoms" as well as an alkoxy group obtained by substituting one or more hydrogen atoms included in the "linear alkoxy group having 1 to 6 carbon atoms" with another or other "linear alkoxy groups having 1 to 6 carbon atoms" that may be the same or different. Examples of such a "linear alkoxy group having 1 to 6 carbon atoms that may have undergone substitution by a linear alkoxy group having 1 to 6 carbon atoms" include a methoxy group, an ethoxy group, an n-propoxy group, a methoxymethoxy group, an ethoxymethoxy group, an n-propoxymethoxy group, a methoxyethoxy group (e.g., a 2-methoxyethoxy group), an ethoxyethoxy group (e.g., a 2-ethoxyethoxy group), and an n-propoxyethoxy group.

The term "cyanoalkoxy group having 1 to 6 carbon atoms" as used herein refers to a group obtained by substituting at least one hydrogen atom included in any linear, branched, or cyclic alkoxy group having 1 to 6 carbon atoms with a cyano group.

The term "linear alkylthio group having 1 to 6 carbon atoms" as used herein encompasses alkylthio groups that include any linear alkyl group having 1 to 6 carbon atoms. Examples thereof include a methythio group, an ethylthio group, and an n-propylthio group. Meanwhile, the term "alkylthio group having 1 to 6 carbon atoms" refers to any linear, branched, or cyclic alkylthio group having 1 to 6 carbon atoms.

The term "linear alkylamino group having 1 to 6 carbon atoms" as used herein encompasses amino groups that include any one or two linear alkyl groups having 1 to 6 carbon atoms. Examples thereof include a methylamino group, a dimethylamino group, an ethylamino group, a methylethylamino group, and a diethylamino group. Meanwhile, the term "dialkylamino group that includes alkyl groups having 1 to 6 carbon atoms" encompasses amino groups that include any two alkyl groups having 1 to 6 carbon atoms.

The term "alkenyl group having 2 to 7 carbon atoms that may be branched or cyclic" as used herein encompasses any linear alkenyl groups having 2 to 7 carbon atoms, any branched alkenyl groups having 3 to 7 carbon atoms, and any cyclic alkenyl groups having 3 to 7 carbon atoms. Such groups may also be referred to merely as "lower alkenyl groups". Examples of any linear alkenyl groups having 2 to 7 carbon atoms include an ethenyl group, a 1-propenyl group, a 2-propenyl group, a 1-butenyl group, a 2-butenyl group, a 1-pentenyl group, a 2-pentenyl group, a 3-pentenyl group, a 4-pentenyl group, and a 1-hexenyl group; examples of any branched alkenyl groups having 3 to 7 carbon atoms include an isopropenyl group, a 1-methyl-1-propenyl group, a 1-methyl-2-propenyl group, a 2-methyl-1-propenyl group, a 2-methyl-2-propenyl group, and a 1-methyl-2-butenyl group; and examples of any cyclic alkenyl groups having 3 to 7 carbon atoms include a cyclobutenyl group, a cyclopentenyl group, and a cyclohexenyl group.

The term "aryl group having n₁ to n₂ carbon atoms" as used herein encompasses any aryl groups having n₁ to n₂ carbon atoms (where n₁ and n₂ are integers and satisfy the relationship n₁<n₂). For example, "aryl group having 6 to 20 carbon atoms" encompass a phenyl group, a naphthyl group, and the like.

The term "heteroaryl group having n₁ to n₂ carbon atoms" as used herein encompasses any heteroaryl groups having n₁ to n₂ carbon atoms (where n₁ and n₂ are integers and satisfy the relationship n₁<n₂) that include one or more heteroatoms. Examples of the heteroatom included in the heteroaryl group include an oxygen atom, a nitrogen atom, and a sulfur atom. For example, "heteroaryl groups having 4 to 20 carbon atoms" encompass heteroaryl groups having 4 to 20 carbon atoms that include one or more heteroatoms as described above.

The term "aryl group having 3 to 10 carbon atoms that may include a heteroatom" as used herein encompasses any aryl groups having 6 to 10 carbon atoms that are constituted by only a hydrocarbon, and any heteroaryl groups having 3 to 12 carbon atoms obtained by substituting at least one carbon atom included in the ring structure of the above-mentioned aryl groups with a heteroatom (e.g., a nitrogen atom, an oxygen atom, and a sulfur atom, and a combination thereof. Examples of the aryl groups having 6 to 10 carbon atoms include a phenyl group, a naphthyl group, an indenyl group, and an azulenyl group; and examples of any heteroaryl groups having 3 to 12 carbon atoms include a pyridyl group, a pyrrolyl group, a quinolyl group, an indolyl group, an imidazolyl group, a furyl group, and a thienyl group.

Examples of the term "aralkyl group with an aryl moiety having 3 to 12 carbon atoms that may include a heteroatom" as used herein include a benzyl group, a phenethyl group, a naphthylmethyl group, a 3-phenylpropyl group, a 2-phenylpropyl group, a 4-phenylbutyl group, a 2-phenylbutyl group, a pyridylmethyl group, an indolylmethyl group, a furylmethyl group, a thienylmethyl group, a pyrrolylmethyl group, a 2-pyridylethyl group, a 1-pyridylethyl group, and a 3-thienylpropyl group.

Examples of the term "acyl group" as used herein include aliphatic acyl groups and aromatic acyl groups. Specifically, examples of the aliphatic acyl groups include alkylcarbonyl groups such as a formyl group, an acetyl group, a propionyl group, a butyryl group, an isobutyryl group, a pentanoyl group, a pivaloyl group, a valeryl group, an isovaleryl group, an octanoyl group, a nonanoyl group, a decanoyl group, a 3-methylnonanoyl group, a 8-methylnonanoyl group, a 3-ethyloctanoyl group, a 3,7-dimethyloctanoyl group, an undecanoyl group, a dodecanoyl group, a tridecanoyl group, a tetradecanoyl group, a pentadecanoyl group, a hexadecanoyl group, a 1-methylpentadecanoyl group, a 14-methylpentadecanoyl group, a 13,13-dimethyltetradecanoyl group, a heptadecanoyl group, a 15-methylhexadecanoyl group, an octadecanoyl group, a 1-methylheptadecanoyl group, a nonadecanoyl group, an eicosanoyl group, and a heneicosanoyl group; carboxylated alkylcarbonyl groups such as a succinoyl group, a glutaroyl group, and an adipoyl group; halogeno lower-alkyl-carbonyl groups such as a chloroacetyl group, a dichloroacetyl group, a trichloroacetyl group, and a trifluoroacetyl group; lower-alkoxy-lower-alkyl-carbonyl groups such as a methoxyacetyl group; and unsaturated alkylcarbonyl groups such as an (E)-2-methyl-2-butenoyl group. Examples of the aromatic acyl groups include arylcarbonyl groups such as a benzoyl group, an α-naphthoyl group, and a β-naphthoyl group; halogeno arylcarbonyl groups such as a 2-bromobenzoyl group and a 4-chlorobenzoyl group; low-alkylated arylcarbonyl groups such as a 2,4,6-trimethylbenzoyl group and a 4-toluoyl group; low-alkoxylated arylcarbonyl groups such as a 4-anisoyl group; carboxylated arylcarbonyl groups such as a 2-carboxybenzoyl group, a 3-carboxybenzoyl group, and a 4-carboxybenzoyl group; nitrated arylcarbonyl groups such as a 4-nitrobenzoyl group and a 2-nitrobenzoyl group; low-alkoxycarbonylated arylcarbonyl groups such as a 2-(methoxycarbonyl)benzoyl group; and arylated arylcarbonyl groups such as a 4-phenylbenzoyl group. A formyl group, an acetyl group, a propionyl group, a butyryl group, an isobutyryl group, a pentanoyl group, a pivaloyl group, and a benzoyl group are favorable.

Examples of the term "silyl group" as used herein include tri-lower-alkyl-silyl groups such as a trimethylsilyl group, a triethylsilyl group, an isopropyldimethylsilyl group, a t-butyldimethylsilyl group, a methyldiisopropylsilyl group, a methyldi-t-butylsilyl group, and a triisopropylsilyl group; and tri-lower-alkyl-silyl groups that have undergone substitution by one or two aryl groups, such as a diphenylmethylsilyl group, a butyldiphenylbutylsilyl group, a diphenylisopropylsilyl group, and a phenyldiisopropylsilyl group. A trimethylsilyl group, a triethylsilyl group, a triisopropylsilyl group, a t-butyldimethylsilyl group, and a t-butyldiphenylsilyl group are favorable, and a trimethylsilyl group is more favorable.

Examples of the term "halogen atom" as used herein include a fluorine atom, a chlorine atom, a bromine atom, and an iodine atom. A fluorine atom or a chlorine atom is favorable.

"Protecting groups" in the terms "amino group protecting group for nucleic acid synthesis", "hydroxy group protecting group for nucleic acid synthesis", "hydroxy group protected by a protecting group for nucleic acid synthesis", "phosphate group protected by a protecting group for nucleic acid synthesis", and "mercapto group protected by a protecting group for nucleic acid synthesis" as used herein are not particularly limited as long as they can stably protect an amino group, a hydroxy group, a phosphate group, or a mercapto group during nucleic acid synthesis. Specifically, the protecting groups are stable under an acidic or neutral condition and can be cleaved using chemical techniques such as hydrogenolysis, hydrolysis, electrolysis, and photolysis. Examples of such protecting groups include lower alkyl groups, lower alkenyl groups, acyl groups, tetrahydropyranyl or tetrahydrothiopyranyl groups, tetrahydrofuranyl or tetrahydrothiofuranyl groups, silyl groups, lower-alkoxy-methyl groups, low-alkoxylated lower-alkoxy-methyl groups, halogeno lower-alkoxy-methyl groups, low-alkoxylated ethyl groups, halogenated ethyl groups, methyl groups that have undergone substitution by 1 to 3 aryl groups, "methyl groups that have undergone substitution by 1 to 3 aryl groups in which an aryl ring has undergone substitution by a lower alkyl group, lower alkoxy group, halogen atom, or cyano group", lower-alkoxy-carbonyl groups, "aryl groups that have undergone substitution by a halogen atom, lower alkoxy group, or nitro group", "lower-alkoxy-carbonyl groups that have undergone substitution by a halogen atom or tri-lower-alkyl-silyl group", alkenyloxycarbonyl groups, and "aralkyloxycarbonyl groups in which an aryl ring may have undergone substitution by a lower alkoxy group or nitro group".

More specific examples of the tetrahydropyranyl groups or tetrahydrothiopyranyl groups include a tetrahydropyran-2-yl group, a 3-bromotetrahydropyran-2-yl group, a 4-methoxytetrahydropyran-4-yl group, a tetrahydrothiopyran-4-yl group, and a 4-methoxytetrahydrothiopyran-4-yl group. Examples of the tetrahydrofuranyl groups or tetrahydrothiofuranyl groups include a tetrahydrofuran-2-yl group and a tetrahydrothiofuran-2-yl group. Examples of the lower-alkoxy-methyl groups include a methoxymethyl group, a 1,1-dimethyl-1-methoxymethyl group, an ethoxymethyl group, a propoxymethyl group, an isopropoxymethyl group, a butoxymethyl group, and a t-butoxymethyl group. An example of the low-alkoxylated lower-alkoxy-methyl groups is a 2-methoxyethoxymethyl group. Examples of the halogeno lower-alkoxy-methyl groups include a 2,2,2-trichloroethoxymethyl group and a bis(2-chloroethoxy)methyl group. Examples of the low-alkoxylated ethyl groups include a 1-ethoxyethyl group and a 1-(isopropoxy)ethyl group. An example of the halogenated ethyl groups is a 2,2,2-trichloroethyl group. Examples of the methyl groups that have undergone substitution by 1 to 3 aryl groups include a benzyl group, an α-naphthylmethyl group, a β-naphthylmethyl group, a diphenylmethyl group, a triphenylmethyl group, an α-naphthyldiphenylmethyl group, and a 9-anthrylmethyl group. Examples of the "methyl groups that have undergone substitution by 1 to 3 aryl groups in which an aryl ring has undergone substitution by a lower alkyl group, lower alkoxy group, halogen atom, or cyano group" include a 4-methylbenzyl group, a 2,4,6-trimethylbenzyl group, a 3,4,5-trimethylbenzyl group, a 4-methoxybenzyl group, a 4-methoxyphenyldiphenylmethyl group, a 4,4'-dimethoxytriphenylmethyl group, a 2-nitrobenzyl group, a 4-nitrobenzyl group, a 4-chlorobenzyl group, a 4-bromobenzyl group, and a 4-cyanobenzyl group. Examples of the lower-alkoxy-carbonyl groups include a methoxycarbonyl group, an ethoxycarbonyl group, a t-butoxycarbonyl group, and an isobutoxycarbonyl group. Examples of the "aryl groups that have undergone substitution by a halogen atom, lower alkoxy group, or nitro group" include a 4-chlorophenyl group, a 2-fluorophenyl group, a 4-methoxyphenyl group, a 4-nitrophenyl group, and a 2,4-dinitrophenyl group. Examples of the "lower-alkoxy-carbonyl groups that have undergone substitution by a halogen atom or tri-lower-alkyl-silyl group" include a 2,2,2-trichloroethoxycarbonyl group and 2-trimethylsilylethoxycarbonyl group. Examples of the alkenyloxycarbonyl groups include a vinyloxycarbonyl group and an aryloxycarbonyl group. Examples of the "aralkyloxycarbonyl groups in which an aryl ring may have undergone substitution by a lower alkoxy group or nitro group" include a benzyloxycarbonyl group, a 4-methoxybenzyloxycarbonyl group, a 3,4-dimethoxybenzyloxycarbonyl group, a 2-nitrobenzyloxycarbonyl group, and a 4-nitrobenzyloxycarbonyl group.

In an embodiment, examples of the "hydroxy group protecting group for nucleic acid synthesis" include aliphatic acyl groups, aromatic acyl groups, methyl groups that have undergone substitution by 1 to 3 aryl groups, "methyl groups that have undergone substitution by 1 to 3 aryl groups in which an aryl ring has undergone substitution by a lower alkyl, lower alkoxy, halogen, or cyano group", and silyl groups. Alternatively, in an embodiment, examples of the "hydroxy group protecting group for nucleic acid synthesis" include an acetyl group, a benzoyl group, a benzyl group, a p-methoxybenzoyl group, a dimethoxytrityl group, a monomethoxytrityl group, a tert-butyldiphenylsilyl group, a tert-butyldimethylsilyl (TBDMS) group, a [(triisopropylsilyl)oxy]methyl (TOM) group, a [(2-nitrobenzyl)oxy]methyl (NBOM) group, a bis(acetoxyethoxy)methyl ether (ACE) group, a tetrahydro-4-methoxy-2H-pyran-2-yl (Mthp) group, a 1-(2-cyanoethoxy)ethyl (CEE) group, a 2-cyanoethoxymethyl (CEM) group, a tert-butyldithiomethyl (DTM) group, a 2-(4-tolylsulfonyl)ethoxymethyl (TEM) group, and a 4-(N-dichloroacetyl-N-methylamino)benzyloxymethyl (4-MABOM) group.

In an embodiment, examples of the protecting group used for the "hydroxy group protected by a protecting group for nucleic acid synthesis" include aliphatic acyl groups, aromatic acyl groups, "methyl groups that have undergone substitution by 1 to 3 aryl groups", "aryl groups that have undergone substitution by a halogen atom, lower alkoxy group, or nitro group", lower alkyl groups, and lower alkenyl groups. Alternatively, in an embodiment, examples of the protecting group used for the "hydroxy group protected by a protecting group for nucleic acid synthesis" include a benzoyl group, a benzyl group, a 2-chlorophenyl group, a 4-chlorophenyl group, and a 2-propenyl group.

In an embodiment, examples of the "amino group protecting group for nucleic acid synthesis" include acyl groups, and a benzoyl group is favorable.

In an embodiment, examples of the "protecting group" used for the "phosphate group protected by a protecting group for nucleic acid synthesis" include lower alkyl groups, lower alkyl groups that have undergone substitution by a cyano group, aralkyl groups, "aralkyl groups in which an aryl ring has undergone substitution by a nitro group or halogen atom", and "aryl groups that have undergone substitution by a lower alkyl group, halogen atom, or nitro group". Alternatively, in an embodiment, examples of the "protecting group" used for the "phosphate group protected by a protecting group for nucleic acid synthesis" include a 2-cyanoethyl group, a 2,2,2-trichloroethyl group, a benzyl group, a 2-chlorophenyl group, and a 4-chlorophenyl group.

In an embodiment, examples of the "protecting group" used for the "mercapto group protected by a protecting group for nucleic acid synthesis" include aliphatic acyl groups and aromatic acyl groups, and a benzoyl group is favorable.

The term "pharmaceutically acceptable salt thereof" as used herein means a salt of a specific compound that has no risk of entirely or partially inhibiting the purpose of a pharmaceutical application where it is used. Examples of such a salt include metal salts including alkali metal salts such as sodium salts, potassium salts, and lithium salts, alkali earth metal salts such as calcium salts and magnesium salts, aluminum salts, iron salts, zinc salts, copper salts, nickel salts, and cobalt salts; amine salts including inorganic salts such as ammonium salts, and organic salts such as t-octylamine salts, dibenzylamine salts, morpholine salts, glucosamine salts, phenylglycine alkylester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts including halide hydroacid salts such as hydrofluoric acid salts, hydrochloric acid salt, hydrobromic acid salts, and hydroiodic acid salts, nitrates, perchlorates, sulfates, and phosphates; organic acid salts including lower-alkane-sulfonates such as methanesulfonates, trifluoromethanesulfonates, and ethanesulfonates, arylsulfonates such as benzenesulfonates and p-toluenesulfonates, acetates, malates, fumarates, succinates, citrates, tartrates, oxalates and maleates; and amino acid salts such as glycine salts, lysine salts, arginine salts, ornithine salts, glutamates, and aspartates.

The term "pharmaceutically acceptable cation" as used herein means a cation that has no risk of entirely or partially inhibiting, for example, the purpose of a pharmaceutical application where the compound according to the present invention is used, and encompasses, for example, a hydrogen ion, a sodium ion, a potassium ion, an ammonium ion, and the like.

The term "pharmaceutically acceptable anion" as used herein means an anion that has no risk of entirely or partially inhibiting, for example, the purpose of a pharmaceutical application where the compound according to the present invention is used, and encompasses, for example, a chloride ion, a bromide ion, a hydroxide ion, an acetate ion, a carbonate ion, a phosphate ion, and the like.

The term "nucleic acid" as used herein is a generic term for ribonucleic acid (RNA) and deoxyribonucleic acid (DNA), and refers to a biopolymer consisting of nucleotides that are each composed of a base, a sugar, and a phosphate and are linked via phosphodiester bonds. RNA is nucleic acid in which the sugar moiety is ribose, and DNA is nucleic acid in which the sugar moiety is 2-deoxyribose obtained from ribose through substitution of a hydroxy group at position 2' with a hydrogen group. Position 5' of the sugar is linked to position 3' of the adjacent sugar via a phosphodiester bond, and a chain is formed through repeated formation of this bond.

"Nucleosides" include "nucleosides" in which a purine base or a pyrimidine base is linked to a sugar, as well as "nucleosides" in which a heteroaromatic ring or an aromatic hydrocarbon ring other than purine and pyrimidine that can serve as a substitute for a purine or pyrimidine base is linked to a sugar. A nucleoside in a natural form is also referred to as a "natural nucleoside". A nucleoside in a modified and non-natural form is also referred to as a "modified nucleoside", and in particular, a nucleotide in which a sugar moiety is modified is referred to as a "sugar-modified nucleoside". The term "nucleotide" means a compound obtained through binding of a phosphate group to a sugar of a nucleoside. The "nucleosides" and the "nucleotides" include those contained in DNA and those contained in RNA.

The term "oligonucleotide" as used herein refers to a polymer of "nucleotides" in which, for example, five to seventy, preferably ten to forty and more preferably twelve to thirty, of the same or different "nucleosides" are linked via phosphodiester bonds or other bonds, and includes oligonucleotides in a natural form and oligonucleotides in a non-natural form. Favorable examples of the "oligonucleotides" in a non-natural form include sugar derivatives with sugar moieties modified, thioated derivatives with phosphate diester moieties thioated; esters with terminal phosphate moieties esterified; and amides in which amino groups on purine bases are amidated, and the sugar derivatives with sugar moieties modified are more favorable. The "oligonucleotides" may be constituted by natural nucleosides or modified nucleosides, or may be a mixture of natural nucleosides and modified nucleosides.

The bond (inter-nucleoside bond) between the sugars in the oligonucleotide may be a phosphodiester (D-oligo), which is a bond in a natural nucleic acid, or an artificially modified bond (e.g., phosphorothioate (S-oligo), methylphosphonate (M-oligo), or boranophosphonate). Any bonds known in the art can be used. The S-oligo (phosphorothioate) has a PS skeleton obtained by substituting oxygen atoms in the phosphate group moieties in the phosphodiester bonds between the nucleosides with sulfur atoms. This modification is incorporated into oligonucleotides according to a known method. An antisense oligonucleotide (ASO) with this modification at one or more sites per oligonucleotide is also referred to as an S-oligo-type (phosphorothioate-type) antisense oligonucleotide. In the oligonucleotide, all the bonds may be the same, or different bonds may be included. It is preferable that the oligonucleotide of the present invention includes the D-oligo and/or the S-oligo.

Any modification for a nucleotide known in the art can also be used. A sugar modification and a nucleic acid base modification are known as the modification for a nucleotide. These nucleic acid modifications can be provided based on a method known in the art.

Examples of the nucleic acid base modification include 5-methylcytosine, 5-hydroxymethylcytosine, and 5-propynylcytosine.

Examples of the sugar modification include substitution at position 2' of the sugar, and a cross-linked structure between position 4' and position 2' of the sugar. Examples of the substitution at position 2' of the sugar include 2'-F, 2'-OCH₃ (2'-OMe), and 2'-OCH₂CH₂OCH₃ (2'-MOE).

An "alteration" of a nucleic acid refers to any change of nucleic acid, such as substitution, deletion, or insertion of a base, or a change in a chemical structure other than a base (e.g., the modification as described above). In this specification, a substance that includes at least one of such alterations may be referred to as an "altered substance".

### Compound and Pharmaceutically Acceptable Salt Thereof

The compound and pharmaceutically acceptable salt thereof according to the present invention have, for example, a phenothiazine structure or a structure similar to phenothiazine.

The compound according to the present invention is represented by Formula (I) below: 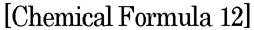 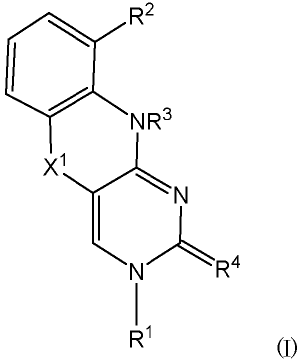 (in Formula (I),
R¹ is a group represented by Formula (II) below: 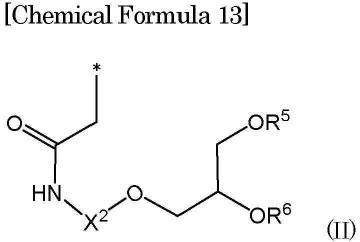 (in Formula (II),
X² is a group represented by the formula below: 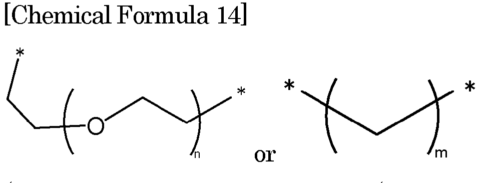 where n is any of integers of 2 to 4 specifically 2, 3, or 4,
m is any of integers of 2 to 4 (specifically 2, 3, or 4), and
* is an atomic bonding),
R⁵ is a hydrogen atom, an alkyl group having 1 to 7 carbon atoms that may be branched or cyclic, or a hydroxy group protecting group for nucleic acid synthesis,
R⁶ represents a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from an α group and may include a heteroatom, an aralkyl group with an aryl moiety having 3 to 12 carbon atoms that may have any one or more substituents selected from the α group and may include a heteroatom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R¹⁴)R¹⁵ (where R¹⁴ and R¹⁵ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms),
the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms, and
* is an atomic bonding),
R² is a hydrogen atom, a hydroxy group, a nitro group, an amino group, or a group represented by Formula (III) below: 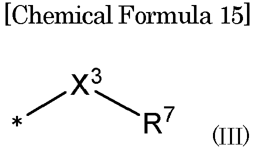 (in Formula (III),
X³ is an oxygen atom or a sulfur atom, and
R⁷ is a group represented by the formula below: 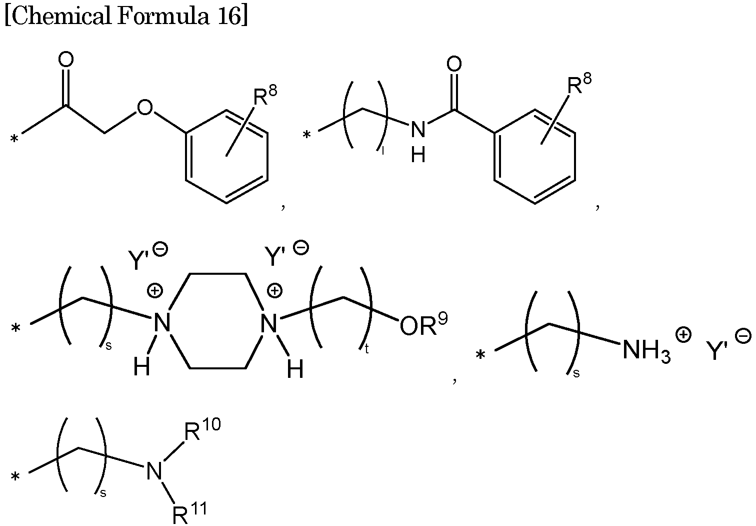 or
   (where
R⁸ is a hydrogen atom, a hydroxy group, an alkyl group having 1 to 7 carbon atoms that may be branched or cyclic, or an alkoxy group having 1 to 7 carbon atoms that may be branched or cyclic,
R⁹ is a hydrogen atom, or an alkyl group having 1 to 7 carbon atoms that may be branched or cyclic,
R¹⁰ and R¹¹ are each independently a hydrogen atom, an alkyl group having 1 to 7 carbon atoms that may be branched or cyclic, or an amino group protecting group,
   Y' is a pharmaceutically acceptable anion,
   s is any of integers of 2 to 4 (specifically 2, 3, or 4),
   t is any of integers of 2 to 4 (specifically 2, 3, or 4),
   l is any of integers of 1 to 5 (specifically 1, 2, 3, 4, or 5), and
   * is an atomic bonding)),
R³ is a hydrogen atom, an alkyl group having 1 to 7 carbon atoms that may be branched or cyclic, or an amino group protecting group,
R⁴ is an oxygen atom or a sulfur atom, and
X¹ is an oxygen atom or a sulfur atom).

In an embodiment, the present invention provides a compound represented by Formula (Ia) below and a pharmaceutically acceptable salt thereof 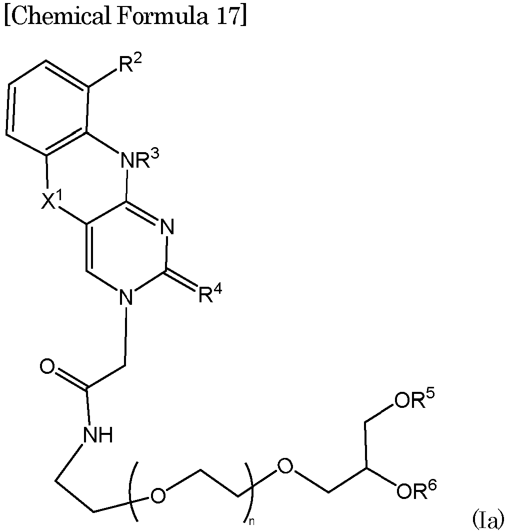 (in Formula (Ia), R², R³, R⁴, R⁵, R⁶, X¹, and n are as defined above).

Alternatively, in an embodiment, the present invention provides a compound represented by Formula (Ib) below and a pharmaceutically acceptable salt thereof 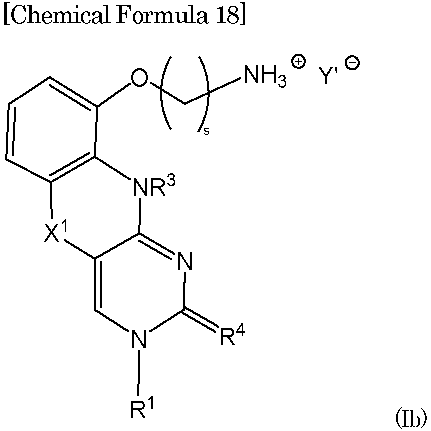 (in Formula (Ib), R¹, R³, R⁴, X¹, Y', and s are as defined above).

Alternatively, in an embodiment, the present invention provides a compound represented by Formula (Ic) below and a pharmaceutically acceptable salt thereof 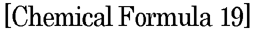 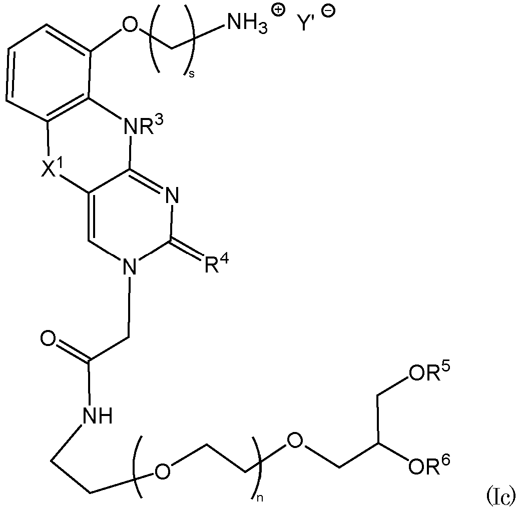 (in Formula (Ic), R³, R⁴, R⁵, R⁶, X¹, Y', n, and s are as defined above).

The compound according to the present invention represented by Formula (I) can be synthesized by, for example, by producing a known compound described in a known reference such as E. Pedroso et al., Org. Lett., 9, 4503-4506 (2007) from 5-bromouracil and performing a plurality of reaction processes using the compound as a starting material.

### Drug Delivery System Formulation

The drug delivery system (which may also be referred to as "DDS" hereinafter) formulation according to the present invention includes, for example, a ligand derived from the compound represented by Formula (I) above or a pharmaceutically acceptable salt thereof.

Alternatively, the DDS formulation of the present invention includes, for example, a ligand derived from a compound represented by Formula (I') below or a pharmaceutically acceptable salt thereof 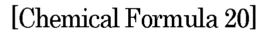 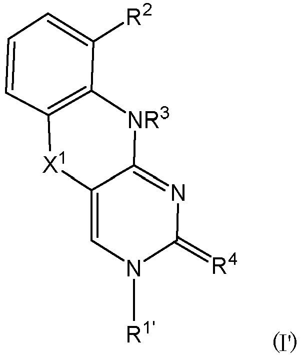 (in Formula (I'),
R^{1'} is
   a hydrogen atom, an alkyl group having 1 to 7 carbon atoms that may be branched or cyclic, an alkoxy group having 1 to 7 carbon atoms that may be branched or cyclic, or an alkylthio group having 1 to 6 carbon atoms that may be branched; or
   a pentose moiety that includes a chemical structure selected from the group consisting of a ribos- 1-yl structure and a 2-deoxyribos-1-yl structure,
R² is a hydrogen atom, a hydroxy group, a nitro group, an amino group, or a group represented by Formula (III) below: 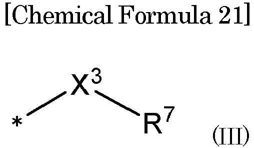 (in Formula (III),
   X³ is an oxygen atom or a sulfur atom, and
   R⁷ is a group represented by the formula below: 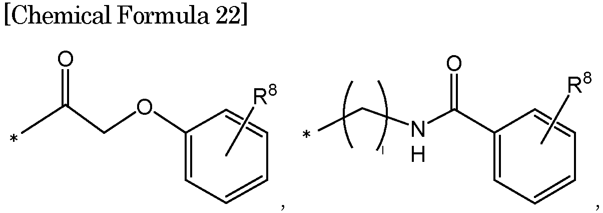 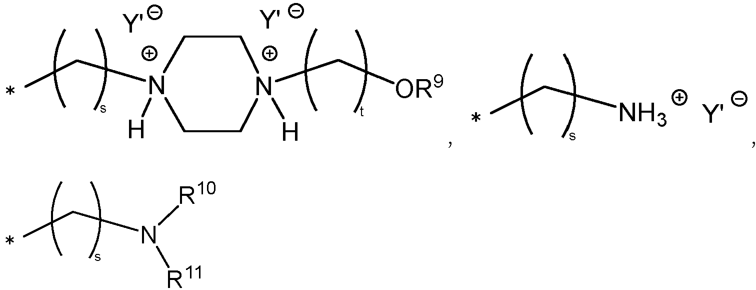 or
      (where
      R⁸ is a hydrogen atom, a hydroxy group, an alkyl group having 1 to 7 carbon atoms that may be branched or cyclic, or an alkoxy group having 1 to 7 carbon atoms that may be branched or cyclic,
      R⁹ is a hydrogen atom, or an alkyl group having 1 to 7 carbon atoms that may be branched or cyclic,
      R¹⁰ and R¹¹ are each independently a hydrogen atom, an alkyl group having 1 to 7 carbon atoms that may be branched or cyclic, or an amino group protecting group,
      Y' is a pharmaceutically acceptable anion,
      s is any of integers of 2 to 4,
      t is any of integers of 2 to 4,
      l is any of integers of 1 to 5, and
      * is an atomic bonding)),
R³ is a hydrogen atom, an alkyl group having 1 to 7 carbon atoms that may be branched or cyclic, or an amino group protecting group,
R⁴ is an oxygen atom or a sulfur atom, and
X¹ is an oxygen atom or a sulfur atom).

In an embodiment, the DDS formulation according to the present invention includes a ligand derived from a compound represented by Formula (I'a) below or a pharmaceutically acceptable salt thereof 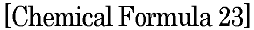 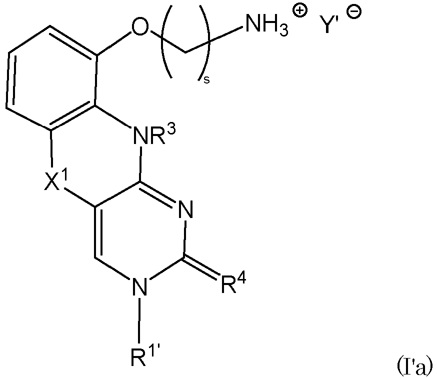 (in Formula (I'a), R^{1'}, R³, R⁴, X¹, Y', and s are as defined above).

Alternatively, in an embodiment of the DDS formulation according to the present invention, R^{1'} in Formula (I') above is a pentose moiety that includes a chemical structure selected from the group consisting of a ribos-1-yl structure and a 2-deoxyribos-1-yl structure. Such a pentose moiety is, for example, a moiety obtained through desorption of a base moiety from at least one monomer selected from the group consisting of a DNA monomer, an RNA monomer, and an artificial nucleic acid monomer. It is preferable that the above-mentioned pentose moiety is a moiety obtained through desorption of a base moiety from an artificial nucleic acid monomer.

Such an artificial nucleic acid monomer is, for example, a 2',4'-BNA/LNA monomer having a structure represented by Formula (IV) below: 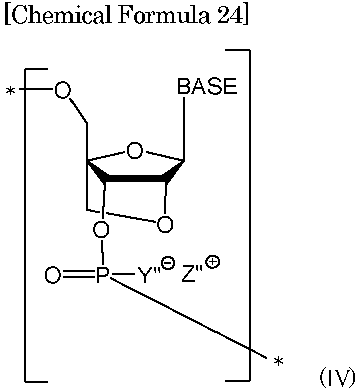 (in Formula (IV),
BASE represents a purin-9-yl group that may have any one or more substituents selected from an α group, or a 2-oxo-1,2-dihydropyrimidin-1-yl group that may have any one or more substituents selected from the α group,
   the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
Y" is an oxygen atom or a sulfur atom,
Z" is a pharmaceutically acceptable cation and,
* is an atomic bonding).

In Formula (IV) above, "BASE" is, for example, a purine base (i.e., a purin-9-yl group) or a pyrimidine base (i.e., a 2-oxo-1,2-dihydropyrimidin-1-yl group). These bases may have any one or more substituents selected from the α group consisting of a hydroxy group, linear alkyl groups having 1 to 6 carbon atoms, linear alkoxy groups having 1 to 6 carbon atoms, a mercapto group, linear alkylthio groups having 1 to 6 carbon atoms, an amino group, linear alkylamino groups having 1 to 6 carbon atoms, and halogen atoms.

Specific examples of "BASE" above include an adeninyl group, a guaninyl group, a cytosinyl group, an uracinyl group, a thyminyl group, a 6-aminopurin-9-yl group, a 2,6-diaminopurin-9-yl group, a 2-amino-6-chloropurin-9-yl group, a 2-amino-6-fluoropurin-9-yl group, a 2-amino-6-bromopurin-9-yl group, a 2-amino-6-hydroxypurin-9-yl group, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl group, a 4-amino-2-oxo-5-ffuoro-1,2-dihydropyrimidin-1-yl group, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl group, a 2-oxo-4-hydroxy-5-methyl-1,2-dihydropyrimidin-1-yl group, and a 4-amino-5-methyl-2-oxo-1,2-dihydropyrimidin-1-yl group.

Alternatively, from the viewpoint of advantageous introduction into a nucleic acid drug, "BASE" is preferably selected from groups represented by the structural formula below: 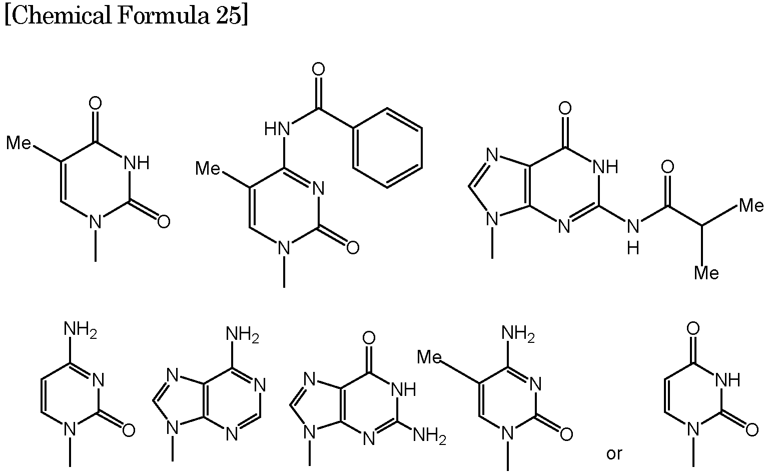 and a 2-oxo-4-hydroxy-5-methyl-1,2-dihydropyrimidin-1-yl group, a 2-oxo-4-amino-1,2-dihydropyrimidin-1-yl group, a 6-aminopurin-9-yl group, a 2-amino-6-hydroxypurin-9-yl group, a 4-amino-5-methyl-2-oxo-1,2-dihydropyrimidin-1-yl group, and a 2-oxo-4-hydroxy1,2-dihydropyrimidin-1-yl group. It is also preferable that a hydroxy group and an amino group included in the above-mentioned groups serving as "BASE" are protected by a protecting group during a reaction with a polynucleic acid, which will be described later.

In an embodiment, the DDS formulation according to the present invention contains the above-mentioned ligand (i.e., the ligand derived from the compound represented by Formula (I) above or a pharmaceutically acceptable salt thereof, or the ligand derived from the compound represented by Formula (I') above or a pharmaceutically acceptable salt thereof in the form of a conjugate where the ligand is linked to a polynucleic acid.

The polynucleic acid that can be included in the conjugate is, for example, an oligonucleotide, and preferably an oligonucleotide that is expected to be delivered to a target viscus in a living body and exhibit desired functions. Examples of the oligonucleotide that is expected to exhibit desired functions include an oligonucleotide that suppresses expression of a target gene, and an oligonucleotide that regulates expression of a target gene.

Such an oligonucleotide encompasses oligonucleotides to be included in nucleic acid drugs. The oligonucleotide may be single-stranded or double-stranded, and specific examples thereof include an antisense oligonucleotide (ASO), siRNA, and a nucleic acid aptamer. The ASO may form a double-stranded oligonucleotide together with a sequence that can bind to a target sequence.

The oligonucleotide is an oligonucleotide with, for example, 5 to 70 bases having a sequence capable of binding to a target sequence in a target gene. The length of the oligonucleotide is preferably 5 or more bases, 6 or more bases, 7 or more bases, 8 or more bases, 9 or more bases, 10 or more bases, 11 or more bases, 12 or more bases, 13 or more bases, 14 or more bases, or 15 or more bases, and 70 or less bases, 50 or less bases, 40 or less bases, 30 or less bases, 25 or less bases, or 20 or less bases.

When the oligonucleotide included in the polynucleic acid is a double-stranded oligonucleotide, the second strand is an oligonucleotide having a sequence capable of binding to the first strand oligonucleotide having a sequence capable of binding to a target sequence in a target gene. The second strand has, for example, 8 to 60 bases, and preferably has 8 or more bases, 9 or more bases, 10 or more bases, 11 or more bases, 12 or more bases, 13 or more bases, 14 or more bases, or 15 or more bases, and 60 or less bases, 50 or less bases, 40 or less bases, 30 or less bases, 25 or less bases, or 20 or less bases. The length of the second strand may be the same as the length of the first strand, or may be shorter than the length of the first strand by a length corresponding to one or several bases as long as the second strand binds to the first strand, or longer than the length of the first strand due to addition of one or several bases to one or two sides of a site that binds to the first strand. Note that the wording "one or several bases" as used herein means, for example, 1 to 10 bases, preferably 1 to 5 bases, more preferably 1 to 3 bases, and even more preferably one or two bases. The favorable length of the second strand depends on the length of the first strand. For example, the favorable length of the second strand is 50% or more, 60% or more, 70% or more, 50 to 100%, 60 to 100%, or 70 to 100% of the length of the first strand.

The wording "the oligonucleotide "binds to" a target sequence" means that a plurality of different single-stranded oligonucleotides or nucleic acids can form a nucleic acid having two or more strands due to complementarity of the nucleic acid bases. This wording preferably means that a double-stranded nucleic acid can be formed. There is no particular limitation on the melting temperature (Tₘ), which is an index of the thermal stability of a bond, of the nucleic acid having two or more strands.

The melting temperature (Tₘ) of double-stranded nucleic acid can be determined, for example, as follows:
Equimolar amounts of an oligonucleotide and a target RNA or DNA are mixed in a phosphate buffer solution (10 mM, pH 7.0) with sodium chloride (final concentration: 100 mM), and the resulting mixture is heated at 100°C and is then allowed to cool slowly to room temperature to form a double-stranded nucleic acid through annealing. The temperature of the double-stranded nucleic acid is raised from 5°C to 90°C at a heating rate of 0.5°C/minute, and the change in absorbance (A) at 260 nm with the temperature (T) is measured. An Avs T graph is drawn based on the measurement results, and the temperature at the point of the intersection of the median line of the tangent line to the pre-transition region and the tangent line to the post-transition region with the A vs T graph is taken as Tₘ of the double-stranded nucleic acid.

The melting temperature (Tₘ) of the double-stranded nucleic acid is, for example, 40°C or higher and preferably 50°C or higher.

The term "complementary" as used herein means that two different single-stranded oligonucleotides or nucleic acids have such a paring relationship that they can form a double-stranded nucleic acid. It is preferable that the base sequences of the regions that form a double-strand is completely complementary to each other, but there may be one or several mismatches therebetween as long as the double-stranded nucleic acid can be formed and a desired function (e.g., suppression or regulation of expression) is exhibited. The term "one or several mismatches" means one to four mismatches, preferably one to three mismatches, and more preferably one or two mismatches, which may depend on the length of the oligonucleotide. The oligonucleotide included in the nucleic acid moiety preferably has 80% or more, 90% or more, 95% or more, 96% or more, 97% or more, 98% or more, or 99% or more complementarity to the base sequence of the region for forming a double-strand, or may be completely (100%) complementary thereto.

The oligonucleotide also encompasses both oligonucleotides containing natural DNA (unmodified oligonucleotides) and oligonucleotides containing chemically modified DNA. Such modifications can alter the activity of the oligonucleotide, and can, for example, improve the affinity for a target nucleic acid or improve the resistance against a nucleolytic enzyme (nuclease). In the present invention, the affinity of the oligonucleotide for a target is improved, thus making it possible to use a shorter oligonucleotide.

The oligonucleotide included in the polynucleic acid can be synthesized according to ordinary methods and can be easily synthesized using, for example, a commercially available automated nucleic acid synthesizer (manufactured by, for example, Applied Biosystems, Gene Design Co., Ltd., or the like). The synthesis methods include a solid-phase synthesis method in which phosphoramidites are used, and a solid-phase synthesis method in which hydrogen phosphonates are used.

In the DDS formulation according to the present invention, when the polynucleic acid included in the conjugate is constituted by an oligonucleotide, the dangling bond of the ligand above may be linked to at least one of the 3' terminus or the 5' terminus of the oligonucleotide and is preferably linked to the 5' terminus.

Furthermore, in the conjugate above, the number of the ligands above linked to a single polylactic acid molecule is preferably one to three and more preferably one. Such a conjugate can be used to deliver the polynucleic acid to the liver as well as a viscus, an organ, or a tissue other than the liver. The viscus, the organ, or the tissue other than the liver is not necessarily limited, but examples thereof include the skeletal muscle, the spleen, the pancreas, the heart, the lung, the stomach, the intestine, the brain, the mammary gland, and the skin. In the present invention, the polynucleic acid is effectively delivered particularly to the skeletal muscle.

### Pharmaceutical

A pharmaceutical according to the present invention contains the above-described drug delivery system formulation.

Regarding the pharmaceutical according to the present invention, there is no particular limitation on the administration method and the dosage form, and an administration method and a dosage form known in the art can be used.

The pharmaceutical according to the present invention can be administered using various methods depending on a topical or systemic treatment or regions to be treated. Topical administration (including ocular instillation, intravaginal administration, intrarectal administration, intranasal administration, and percutaneous administration), oral administration, or parenteral administration may be employed as the administration method. Examples of the parenteral administration include intravenous injection, intravenous instillation, subcutaneous transfusion, intraabdominal transfusion, intramuscular transfusion, pulmonary administration via the airway through aspiration or inhalation.

The pharmaceutical according to the present invention can be topically administered using dosage forms such as a percutaneous patch, ointment, lotion, cream, gel, drops, suppository, spray, liquid medicine, and powder medicine.

Examples of compositions for oral administration include powder medicine, granular medicine, a suspension or solution obtained through dissolution in water or a non-aqueous medium, a capsule, powdered medicine, and a tablet. Examples of compositions for parenteral administration include sterile aqueous solutions containing a buffer, a diluent, and other appropriate additives.

The pharmaceutical according to the present invention can be obtained by mixing, as necessary, various additives for a drug such as a vehicle, a binder, a humectant, a disintegrator, a lubricant, and a diluent that are suitable for the dosage form to an effective amount of the "polynucleic acid" included in the conjugate in the drug delivery system formulation above. An injection can be made into a formulation through sterilization together with an appropriate carrier.

The administration target individuals (living organisms) are preferably mammals, more preferably humans, monkeys, pet animals such as dogs and cats, and livestock such as cattle and pigs, and even more preferably humans. Although the effective administration dose depends on the individual to which the pharmaceutical is to be administered, the effective administration dose can be determined as desired in accordance with the type, sex, age, weight, symptom and the like of the individual, and the method, route, frequency and the like of the administration.

### Examples

Hereinafter, the present invention will be described using examples, but the present invention is not limited to the examples.

The apparatuses used in the following examples were as follows.

¹H-NMR, ¹³C-NMR, ¹⁹F-NMR, and ³¹P-NMR were recorded using spectrometers JNM-ECS400 and JNM-ECA500 manufactured by JEOL Ltd. The chemical shift values in ¹H-NMR are reported in parts per million (ppm) compared to methanol-d₄ (3.31 ppm) or acetonitrile-da (1.93 ppm). The chemical shift values in ¹³C-NMR are reported in ppm relative to methanol-d₄ (49.0 ppm) or acetonitrile-ds (1.32 ppm, 118.3 ppm). Furthermore, the chemical shift values in ³¹P-NMR and ¹⁹F-NMR are reported in ppm relative to external standards 5% H₃PO₄ (0.00 pm) and ethyl trifluoroacetate (-78.7 ppm), respectively.

The nS-8 oligonucleotide synthesizer manufactured by Gene Design was used as an automated DNA synthesizer (for 1.0 pmol scale). A UV melting test was conducted using spectrometers UV 1650PC and UV 1800 manufactured by Shimadzu Corporation that were equipped with a Tₘ analysis accessory quartz cuvette having a 1-cm optical path. IR spectra were recorded using lRAffinity-1S manufactured by Shimadzu Corporation with an NaCl plate. A spectrometer UV-1800 manufactured by Shimadzu Corporation was used to measure UV absorbance. A fluorometer FP-8500 manufactured by JASCO Corporation was used to measure fluorescence spectra. The MALDI-TOF-Mass results from all the new compounds were recorded using SpiralTOF JMS-S3000 manufactured by JEOL Ltd. The MALDI-TOF-Mass results from all the oligonucleotides were recorded using a mass spectrometer Autoflex maX TOF/TOF manufactured by Bruker Daltonics.

Silica gel PSQ-100B or PSQ-60B manufactured by Fuji Silysia Chemical Ltd. was used for column chromatography. CBM-20A, DGU-20A3, LC-20AT, CTO-20A, SPD-20A, and FRC-10A manufactured by Shimadzu Corporation were used for high-performance liquid chromatography (HPLC). In addition, Waters XBridgesTM OST C18 2.5 µm (4.6 × 50 mm) was used for HPLC analysis and Waters XBridges^{™} OST C18 2.5 µm (10 × 50 mm) was used as a preparative column.

The abbreviations of compounds, solvents, and protecting groups described in the following examples are as shown in Table 1.

**Table 1**

| Abbreviation | Name | Abbreviation | Name |
|---|---|---|---|
| ACN | Acetonitrile | ETFA | Ethyl trifluoroacetate |
| AcOEt | Ethyl Acetate | GalNac | *N*-Acetyl-D-galactosamine |
| Cbz | Benzyloxycarbonyl (group) | TEA | Ethylamine |
| DBU | 1,8-Diazabicyclo[5.4.0]undese-7-ene | TFA | Trifluoroacetic Acid |
| DCM | Dichloromethane | TFAA | Trifluoroacetic Anhydride |
| DIAD | Diisopropyl Azodicarboxylate | TFAc | Trifluoroacetyl (group) |
| DIPEA | *N*,*N*-Diisopropylethylamine | THF | Tetrahydrofuran |
| DMF | *N,N*-Dimethylformamide | TMS | Trimethylsilyl (group) |
| DMSO | Dimethyl sulfoxide | Tris | Tris(hydroxymethyl)aminomethane |
| DMTr | 4,4'-Dimethoxytrityl (group) | Ts | *p*-Toluenesulfonyl (group) |
| DMTrCl | 4,4'-Dimethoxy Trityl Chloride | TsCl | *p*-Toluenesulfonyl Chloride |
| | | TSTU | *N,N,N',N'*-Tetramethyl-*O*-(*N*-Succinimidyl) uranium Tetrafluoroborate |

### Example 1: Synthesis of G-clamp Linker (Compound 18)

### (1-1) Synthesis of Compound 7

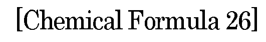

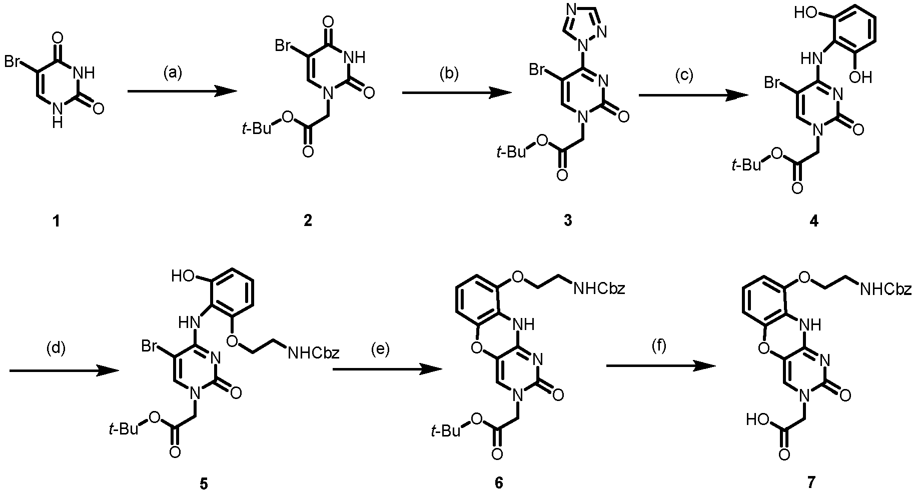

Reagents and conditions for each step: (a) *tert*-butyl bromoacetate, K₂CO₃, DMF, rt, 9 h, 76%. (b) 1,2,4-triazole, POCl₃, TEA, ACN, rt, 20 h, 84%. (c) 2-aminoresorcinol, DBU, ACN, rt, 6 h, 83%. (d) N-Cbz-ethanolamine, PPh₃, DIAD, THF, rt, 19 h, 66%. (e) KF, EtOH, reflux, 66 h, 65%. (f) TFA, DCM, rt, 20.5 h, 91%.

Compound 7 was obtained from 5-bromouracil 1 via Compounds 2 to 6 using the method described in E. Pedroso et al., Org. Lett., 9, 4503-4506 (2007).

### (1-2) Synthesis of Compound 14

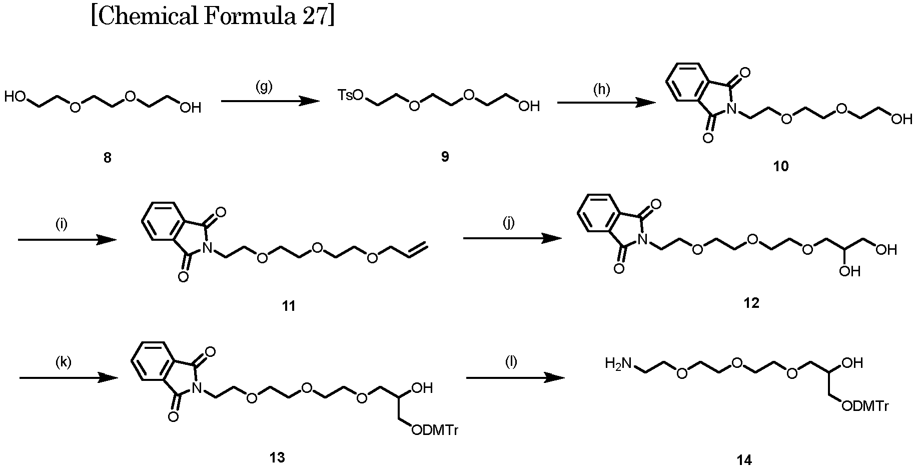

Reagents and conditions for each step: (g) TsCl, pyridine/DCM (1:1), rt, 18 h, 40%. (h) phthalimide, DBU, DMF, reflux, 17 h, 89%. (i) allyl bromide, NaH, THF/DMF (4:1), rt, 24 h, 80%. (j) potassium osmate(VI) dihydrate, *N*-methylmorpholine-*N*-oxide, H₂O/acetone (5:2), it, 26 h, 83%. (k) DMTrCl, pyridine, rt, 4.5 h, 88%. (1) 40% aq. MeNH₂, MeOH, reflux, 8 h, 99%.

Compound 14 was obtained from Compound 8 via Compounds 9 to 13 using J. Chattopadhyaya et al., Helv. Chemica Acta, 82, 2186-2200 (1999).

### (1-3) Synthesis of Compound 18

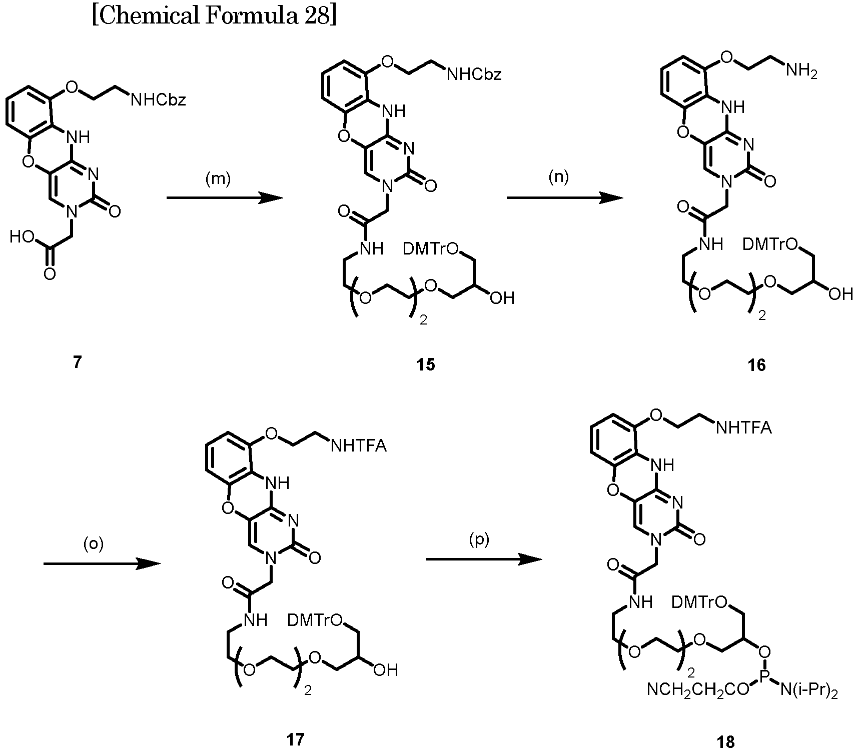

Reagents and conditions for each step: (m) TSTU, DIPEA, DMF, rt, 3.5 h, then compound 14, DMF, it, 30 min, 84%. (n) Pd/C (10%), THF/MeOH (7:1), rt, 2.5 h. (o) ETFA, TEA, MeOH, rt, 24 h, 60% (2 steps). (p) 2-cyanocthyl-,*N,N-*diisopropylchlomphosphoramidite, DIPEA, DCM, rt, 3 h, 78%.

### (1-3-1) Synthesis of Compound 15 (N-(2-(2-(2-(3-(4,4'-dimethoxytrityl)oxy-2-hydropropoxy)ethoxy)ethoxy)ethyl)-2-(9-(N-C bz-2-aminoethoxy)-1,3-diaza-2-oxaphenoxazin-3-yl)acetamide)

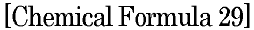

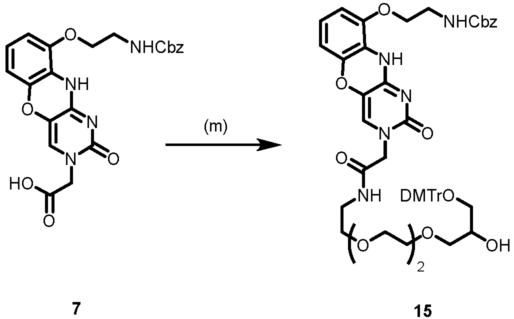

To a suspension of Compound 7 (457 mg, 1.01 mmol) in dry DMF (2 mL), N,N-diisopropylethylamine (DIPEA) (0.35 ml, 2.0 mmol) and N,N,N',N'-tetramethyl-O-(N-succinidyl)uranium tetrafluoroborate (TSTU) (305 mg, 1.01 mmol) were added, and the resulting mixture was stirred at room temperature for 3.5 hours under nitrogen atmosphere. To this mixture, a solution of Compound 14 (613 mg, 1.17 mmol) in dry DMF (2.5 mL) was added, and the resulting mixture was stirred at room temperature for 30 minutes under nitrogen atmosphere. A portion of the solvent was removed under reduced pressure, extraction with CHCl₃ was performed. The organic layer was washed with a saturated aqueous solution of NaHCOs, dried using Na₂SO₄, and concentrated under reduced pressure. The crude product was purified through silica gel column chromatography (MeOH / CHCl₃ = 0 to 3% (triethylamine (TEA 1%)), and thus Compound 15 (815 mg, 84%) was obtained as a white solid. Data on the physical properties of Compound 15 are shown in Table 2.

**Table 2**

| Physical property data of the obtained compound 15 |
|---|
| Mp: 113 °C; IR νₘₐₓ (NaCl plate): 1033,1092,1134,1177,1251,1281,1367,1424,1475, 1504, 1509, 1556, 1561, 1611, 1681, 2877, 2937, 3068, 3294, 3483 cm⁻¹; ¹HNMR(400 MHz, CD₃OD): δ3.13 (2H, d, *J* = 5.5 Hz), 3.38 (2H, t, *J =* 5.3 Hz), 3.50-3.63 (14H, m), 3.75 (6H, s), 3.86-3.91 (1H, m), 4.04 (2H, t, *J =* 5.0 Hz), 4.32 (2H, s), 5.10 (2H, s), 6.39 (1H, d, *J* = 8.3 Hz), 6.58 (1H, d, *J* = 8.3 Hz), 6.78-6.84 (5H, m), 7.09 (1H, s), 7.17 (1H, d, *J* = 7.5 Hz), 7.22-7.38 (11H, m), 7.43 ppm (2H, d, *J* = 7.3 Hz); ¹³C NMR (101 MHz, CD₃OD): δ40.6, 412, 52.7, 55.7, 65.9, 67.6, 69.6, 70.4, 71.1, 712, 71.6, 71.7, 74.1, 87.3, 108.3, 109.4, 114.0, 124.9, 127.8, 128.7, 128.8, 129.0, 129.4, 129.4, 131.3, 137.5, 146.6, 159.1, 160.1, 169.7 ppm ; HR-MS (MALDI-TOF-Mass) Calcd for C₅₂H₅₇N₅O₁₃Na [M+Na]⁺: 982.3851, Found: 982.3856. |

### (1-3-2) Synthesis of Compound 16 (N-(2-(2-(2-(3-(4,4'-dimethoxytrityl)oxy-2-hydroxypropoxy)ethoxy)ethoxy)ethyl)-2-(9-(2-aminoethoxy)-1,3-diaza-2-oxophenoxazm-3-yl)acetamide)

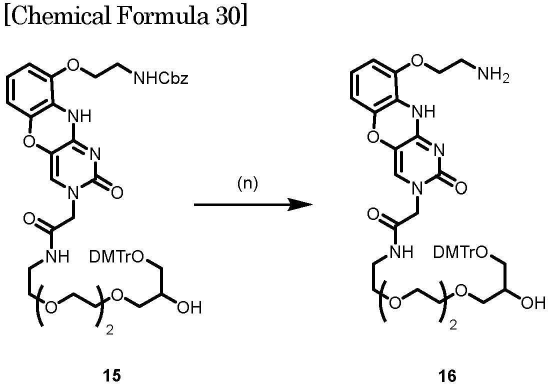

To a solution of Compound 15 obtained above (501 mg, 0.522 mmol) in dry THF (3.4 mL) and MeOH (0.50 mL), Pd/C (10%) (300 mg) was added, and the resulting mixture was vigorously stirred at room temperature for 2.5 hours under hydrogen atmosphere. This mixture was filtered to remove Pd/C and was concentrated under reduced pressure, and thus crude product of Compound 16 was obtained. This product was used in the next reaction without being purified.

### (1-3-3) Synthesis of Compound 17 (N-(2-(2-(2-(3-(4,4'-dimethoxytrityl)oxy-2-hydroxypropoxy)ethoxy)ethoxy)ethyl)-2-(9-(N-trifluoroacetyl-2-aminoethoxy)-1,3-diaza-2-oxophenoxazin-3-yl)acetamide)

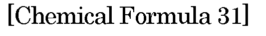

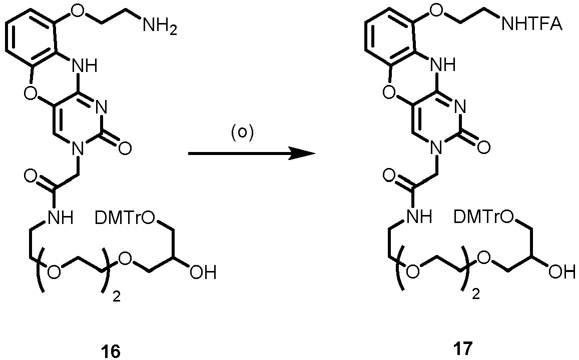

To a solution of the crude product of Compound 16 obtained above in dry MeOH (5.2 mL), dry TEA (0.74 ml, 5.2 mmol) and ethyl trifluoroacetate (ETFA) (0.62 ml, 5.2 mmol) were added, and the resulting mixture was stirred at room temperature for 24 hours under nitrogen atmosphere. To this mixture, TEA (0.58 mL) was added, and the resulting mixture was concentrated under reduced pressure. The crude product was purified through silica gel column chromatography (MeOH / AcOEt = 0 to 5% (TEA 0.5%)), and thus Compound 17 (289 mg, 60% (2 processes)) was obtained as a white solid. Data on the physical properties of Compound 17 are shown in Table 3.

**Table 3**

| Physical property data of the obtained compound 17 |
|---|
| Mp: 92 °C; IR vₘₐₓ (NaCl plate): 1034, 1093,1155, 1179, 1209,1251, 1282, 1369, 1421, 1469, 1478, 1494, 1503, 1510, 1556, 1561, 1611, 1630, 1640, 1677, 1712, 1720, 2881, 2932,3076,3301,3432 cm⁻¹; ¹HNMR (400 MHz, CD₃CN): δ3.02 (2H, d, *J* = 5.4 Hz), 3.24-3.34 (2H, m), 3.43-3.56 (12H, m), 3.63-3.72 (2H, m), 3.74 (6H, s), 3.84 (1H, s), 4.07 (2H, t, *J =* 4.9 Hz), 4.21 (2H, s), 6.41 (1H, d, *J =* 8.3 Hz), 6.53 (1H, d, *J =* 8.4 Hz), 6.81 (5H, td, *J =* 9.0, 8.4 Hz), 6.98 (1H, s), 7.01 (1H, t, *J =* 5.6 Hz), 7.20 (1H, t, *J =* 6.7 Hz), 7.24-7.34 (6H, m), 7.43 (2H, d, *J* = 7.2 Hz), 7.92 (1H, s), 8.10 ppm (1H, s); ¹³C NMR (126 MHz, CD₃CN): δ 39.9, 40.2, 52.5, 55.9, 65.9, 68.0, 70.1, 70.5, 70.9, 71.1, 71.1, 71.3, 73.7, 86.7, 107.7, 109.1, 113.9, 116.0, 116.5, 120.6, 124.3, 127.7, 128.0, 128.7, 129.0, 129.5, 131.0, 137.1, 143.5, 146.3, 146.9, 155.0, 156.2, 157.8, 158.1, 158.4, 158.6, 159.5, 168.7 ppm; ¹⁹F NMR (376 MHz, CD₃CN): δ -77.5 ppm; HR-MS (MALDI-TOF-Mass) Calcd for C₄₆H₅₀N₅O₁₂F₃Na [M+Na]⁺: 944.3306, Found: 944.3309. |

### (1-3-3) Synthesis of Compound 18 (N-(2-(2-(2-(2-(2-cyanoethoxy(diisopropylamino)phosphinoxy)-3-(4,4'-dimethoxytrityl)ox y-propoxy)ethoxy)ethoxy)ethyl)-2-(9-(N-trifluoroacetyl-2-aminoethoxy)-1,3-diaza-2-oxo phenoxazin-3-yDacetamide)

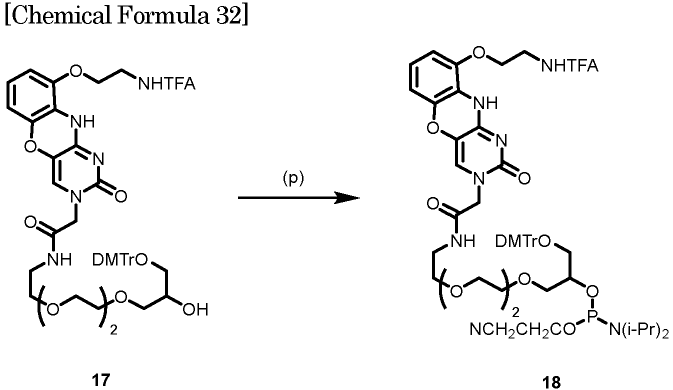

To an ice-cold solution of Compound 17 obtained above (105 mg, 0.114 mmol) in dry dichloromethane (DCM) (1.5 mL), DIPEA (40 pl, 0.23 mmol) and 2-cyanoethyl-N,N-diisopropylchlorophosphoramidite (33 µL, 0.15 mmol) were added, and the resulting mixture was stirred at room temperature for 3 hours under argon atmosphere. The solvent was removed under reduced pressure, and extraction with CHCl₃ was performed. The organic layer was washed with a saturated aqueous solution of NaHCOs, dried using Na₂SO₄, and concentrated under reduced pressure. The crude product was purified through silica gel column chromatography (acetonitrile (ACN) / CHCl₃ = 1 / 1 (TEA 1%)), and thus Compound 18 (100 mg, 78% was obtained as a white solid. Data on the physical properties of Compound 18 are shown in Table 4.

**Table 4**

| Physical property data of the obtained compound 18 |
|---|
| Mp: 64 °C;IR vₘₐₓ (NaCl plate): 1034,1095,1156,1180,1205,1252,1285,1365,1424, 1466, 1478, 1505, 1510, 1556, 1562, 1611, 1633 1639, 1681, 1716, 2250, 2838, 2876, 2934,2966,3064,3258,3553 cm⁻¹; ¹HNMR (400 MHz, CD₃CN): δ1.02 (3H, d, *J* = 6.8 Hz), 1.06-1.24 (9H, m), 2.50 (1H, t, *J =* 6.0 Hz), 2.55-2.73 (1H, m), 3.00-3.06 (0.5H, m), 3.09-3.21 (1.5H, m), 3.24-3.35 (2H, m), 3.40-3.71 (17H, m), 3.73 (6H, s), 3.74-3.90 (1H, m), 4.04 (3H, t, *J =* 5.0 Hz), 4.31 (2H, s), 6.35 (1H, d, *J* = 7.4 Hz), 6.48 (1H, d, *J* = 8.4 Hz), 6.74 (1H, t, *J* = 8.3 Hz), 6.80-6.87 (4H, m) 7.01 (1H, s), 7.12-7.23 (2H, m), 7.24-7.36 (6H, m), 7.40-7.49 (2H, m), 8.03 (1H, s), 8.38 ppm (1H, s); ¹³C NMR (126 MHz, CD₃CN): δ 20.9, 21.0, 21.0, 21.1, 24.9, 24.9, 24.9, 25.0, 25.0, 39.9, 40.1, 43.8, 43.8, 43.9, 43.9, 52.5, 55.9, 59.3, 59.3, 59.4, 59.5, 65.0, 65.0, 68.1, 70.0, 70.9, 70.9, 71.1, 71.1, 71.1, 71.5, 71.6, 72.6, 72.6, 72.7, 72.7, 73.3, 73.4, 86.7, 86.8, 107.8, 109.2, 114.0, 116.0, 116.6, 119.5, 119.8, 124.3, 127.7, 128.0, 128.8, 129.0, 129.0, 129.4, 131.0, 131.0, 131.0, 137.0, 137.1, 137.1, 143.5, 146.2, 146.3, 146.9, 155.1, 156.0, 157.8, 158.1, 158.4, 158.7 159.6, 168.5 ppm;¹⁹F NMR (376 MHz, CD₃CN): δ -77.5 ppm; ³¹P NMR (162 MHz, CD₃CN): δ 149.3,149.8 ppm; HR-MS (MALDI-TOF-Mass) Calcd for C₅₅H₆₇N₇O₁₃F₃Na [M+Na]⁺: 1144.4379, Found: 1144.4401. |

### Example 2: Synthesis and Identification of G clamp Linker-Conjugated Oligonucleotide (ODN)

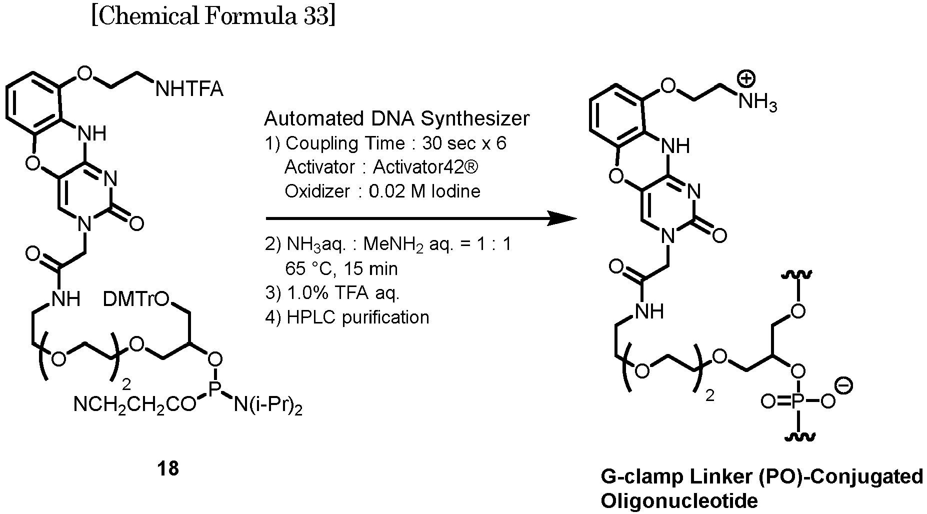

The terms "G-clamp linker moiety", "G-clamp linker (PO)", and "G-clamp linker (PS)" as used herein respectively refer to the following structures.

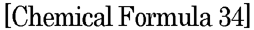

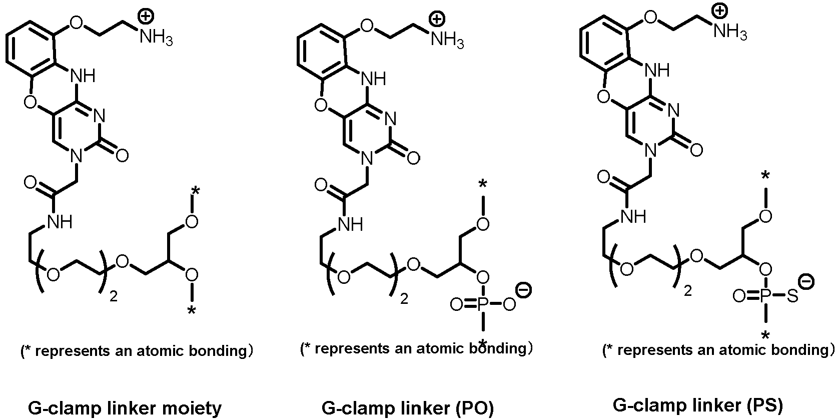

A G-clamp linker (PO)-conjugated oligonucleotide was synthesized from Compound 18 obtained above on a 1.0-µmol scale using an automated DNA synthesizer (nS-8 Oligonucleotides Synthesizer manufactured by GeneDesign). Then, 5-[3,5-bis(trifluoromethyl)phenyl]-1H-tetrazole (Activator 42 (registered trademark)) was used as an activator in all coupling processes. Note that aqueous iodine was used as an oxidizing agent. Compound 18 and natural forms were prepared at a concentration of 100 mM in an ACN solution. The coupling times for Compound 18 and the natural forms were 180 seconds and 30 seconds, respectively. Oligonucleotide synthesis was performed in the DMTr-on mode. The oligonucleotide supported by a solid (CPG) was cleaved, and the protecting groups for the nucleic acid bases and the phosphorodiester bond were removed at 65°C for 15 minutes using a mixed solution (an aqueous solution of methylamine (40%) and an aqueous solution of ammonia (28%) (volume ratio = 1:1)).

DMTr was removed from the crude oligonucleotide having a DMTr group, and the oligonucleotide was roughly purified using a simple reverse-phase column (Waters Sep-Pak (registered trademark) Plus C18 Environmental Cartridges). Specifically, the crude oligonucleotide was added to the Sep-Pak, and then the column was washed using a 10% aqueous solution of ACN. Subsequently, DMTr was removed using a 1% aqueous solution of trifluoroacetic acid, and the oligonucleotide was eluted using a 40% aqueous solution of MeOH. Next, the oligonucleotide was purified through HPLC with an XBridge^{™} OST C18 2.5 pm (10 × 50 mm) reverse-phase HPLC column manufactured by Waters using a gradient elution method in which 100 mM triethylammonium acetate (TEAA) buffer (pH=7.0) was used as an eluent A and MeOH was used as an eluent B.

The components of the purified oligonucleotide were examined through reverse-phase HPLC with XBridge^{™} OST C18 2.5 pm (4.6 × 50 mm) manufactured by Waters and mass spectroscopy with a MALDI-TOF mass spectrometer. The overall yield was calculated from the UV absorbance at 260 nm. Note that the measurement method and the conditions used for the reverse-phase HPLC were as shown in Table 5 below

**Table 5**

| General Conditions |
|---|
| Preparative chromatography: Waters XBridge^{™} OST C18 2.5µm (10×50mm) |
| Preparative chromatography: Waters XBridge^{™} OST C18 2.5µm (4.6×50 mm) |
| Flow rate of preparative conditions: 4.0mL/min |
| Flow rate of preparative conditions: 1.0mL/min |
| Wavelength of UV for oligonucleotide detection: 260nm |
| Column oven temperature: 50°C |
| Eluent A: 100 mM TEAA (pH 7.0) buffer |
| Eluent B: MeOH |
| MeOH gradient in both preparative and analytical conditions (20 min) |
| Retention time (Rt) (min) (Analytical column conditions); |
| 5-70%; ODN 1_X (Rt:9.0min), ODN 2_X (Rt: 10.1min), ODN 3_X(Rt:8.6min), ODN 4_X (Rt:7.8min) |

The results are shown in Table 6.

**Table 6**

| ODN | Sequence (5'-3') | MALDI-TOF-Mass | |
|---|---|---|---|
| | | Calculated [M-H]⁻ | Found [M-H]⁻ |
| **1X** | 5'-d(XGCGTTTTTTGCT)-3' (SEQ ID NO. 1) | 4217.9 | 4218.9 |
| **2X** | 5'-d(XXXGCGTTTTTTGCT)-3' (SEQ ID NO. 2) | 5388.9 | 5390.7 |
| **3X** | 5'-d(GCGTTXTTTGCT)-3' | 3913.7 | 3914.6 |
| **4X** | 5'-d(GCGTCXCTTGCT)-3' | 3883.7 | 3884.9 |

| | | | |
|---|---|---|---|
| X: G-clamp linker (P O) | | | |

### Example 3: Evaluation of Physical Properties of G clamp Linker-Conjugated Oligonucleotide (ODN)

### (1) Evaluation of Double-Strand Forming Ability

Test strands that included, out of the oligonucleotides produced in Example 2, oligonucleotides (ODN 1X and ODN 2X) in which the G-clamp linkers (PO) shown in Table 6 had been introduced to their termini were evaluated for binding affinity for target strands (single-stranded oligo-DNAs and single-stranded oligo-RNAs) through melting temperature measurement.

Specifically, oligonucleotides having the following sequences were synthesized and purified:
5'-N-d(GCGTTTTTTGCT)-3' (SEQ ID NOs: 1, 2, and 5)
(1) N = none (SEQ ID NO: 3)
(2) N = X (X = G-clamp linker (PO)) (SEQ ID NO: 1)
(3) N = XXX (X = G-clamp linker (PO)) (SEQ ID NO: 2)

Note that, in this example, single-stranded oligonucleotides with a 4-base overhang protruding at the 3' terminus were used as complementary strands on the assumption of actual use for delivery, and it was examined how the double-strand forming ability changed depending on the number and positions of guanines. Specifically, single-stranded oligo DNA 3'-d(YYYYCGCAAAAAACGA)-5' (SEQ ID NOs: 4 to 9)
(1) YYYY = none (SEQ ID NO: 4)
(2) YYYY = CATA (SEQ ID NO: 5)
(3) YYYY = CATG (SEQ ID NO: 6)
(4) YYYY = CAGT (SEQ ID NO: 7)
(5) YYYY = CTGG (SEQ ID NO: 8)
(6) YYYY = CGGT (SEQ ID NO: 9), and
   single-stranded oligo RNA 3'-r(YYYYCGCAAAAAACGA)-5' (SEQ ID NOs: 10 to 15)
   (1) YYYY = none (SEQ ID NO: 10)
   (2) YYYY = CAUA (SEQ ID NO: 11)
   (3) YYYY = CAUG (SEQ ID NO: 12)
   (4) YYYY = CAGU (SEQ ID NO: 13)
   (5) YYYY = CUGG (SEQ ID NO: 14)
   (6) YYYY = CGGU (SEQ ID NO: 15)
   were used as the target strands, and the double-strand forming ability (binding affinity) of each oligonucleotide was examined.

The double-strand forming ability of the oligonucleotides was examined by subjecting the different types of oligonucleotides and the target strands to an annealing treatment to form double strands, and then measuring their Tₘ values. More specifically, a sample solution of each oligonucleotide (final concentration: 2 µM) in a phosphate buffer (10 mM, pH 7.0) containing sodium chloride (final concentration: 100 mM) was heated at 100°C and then slowly cooled to room temperature. After that, the sample solution was cooled to 5°C under nitrogen stream before starting the measurement. The temperature was raised to 90°C at a rate of 0.5°C/minute while absorbance at 260 nm was plotted at intervals of 0.5°C. The Tₘ value was calculated using a median method, and a mean value of three independent measurements was adopted. The results are shown in Table 7.

**Table 7**

| 3'-YYYY-5' | *T*ₘ (Δ*Tₘ* = *T*ₘ (X:1 or X:3) - *T*ₘ (X:0)) (°C) | | | | | |
|---|---|---|---|---|---|---|
| | DNA | | | RNA | | |
| | ODN 5 | ODN 1X | ODN2X | ODN 5 | ODN 1X | ODN2X |
| | N = none | N=X | N=XXX | N = none | N=X | N=XXX |
| No YYYY | 38 | 40 (+2) | 31 (-7) | 34 | 35 (+1) | 29 (-5) |
| CAT(U)A | 40 | 42 (+2) | 31 (-9) | 37 | 34 (-3) | 27 (-10) |
| CAT(U)G | 42 | 41 (-1) | 39 (-3) | 38 | 37 (-1) | 33 (-5) |
| CAGT(U) | 38 | 40 (+2) | 34 (-4) | 32 | 31 (-1) | 31 (-1) |
| CT(U)GG | 39 | 44 (+5) | 46 (+7) | 38 | 39 (+1) | 39 (+1) |
| CGGT(U) | 37 | 41 (+4) | 40 (+3) | 34 | 34 (±0) | 35 (+1) |

| | | | | | | |
|---|---|---|---|---|---|---|
| ODN 5: 5'-d(GCGTTTTTTGCT)-3' X = G-clamp linker (P O) | | | | | | |

As shown in Table 7, when the oligonucleotide (ODN 1X) with a single G-clamp linker (PO) introduced to the 5' terminus was used, the Tₘ values of all the target oligo-RNAs remained substantially the same irrespective of the presence or absence and the sequence of the overhang (ΔTₘ: -3°C to 1°C) relative to the case of using the oligonucleotide (ODN5) with no G-clamp linker introduced (N = none). In contrast, when ODN 1X was used, the Tₘ values of the target single-stranded oligo-DNAs with CTGG or CGGT in the overhang moiety significantly increased (ΔTₘ: +4°C to +5°C) relative to the case of using ODN5.

When the oligonucleotide (ODN 2X) with three G-clamp linkers (PO) introduced to the 5' terminus was used, the Tₘ values of the target oligo-RNAs with CAGU, CUGG, or CGGU in the overhang moiety remained substantially the same (ΔTₘ : -1°C to +1°C) relative to the case of using ODN5. Meanwhile, when ODN 2X was used, the Tₘ values of the other target oligo-RNAs significantly decreased (ΔTₘ: -10°C to -5°C) relative to the case of using ODN5. Furthermore, when ODN 2X was used, the Tₘ values of the target oligo-DNAs with CGGT or CTGG in the overhang rose (ΔTₘ: +3°C to +7°C) relative to the case of using ODN5. On the contrary, when ODN 2X was used, the Tₘ values of the other target oligo-DNAs significantly decreased (ΔTₘ: -9°C to -3°C). These results show that the G-clamp linker (PO) introduced to the 5' terminus of the ODN has a notable interaction with none of the overhang sequences during the formation of a double-strand with the target oligo-RNA, whereas the G-clamp linker above interacts with a specific sequence having a guanine in the overhang region during the formation of the double-strand with the target oligo-DNA and improves the double-strand forming ability.

### (2) Evaluation of Fluorescence Characteristics

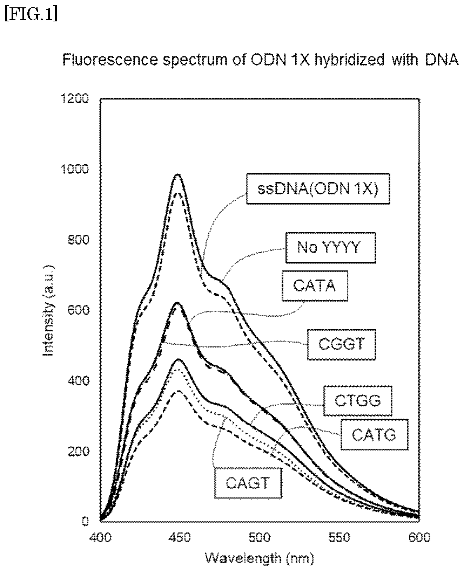

Nucleic acid with a phenoxazine artificial base is known to be excited at 360 nm and produce fluorescence at 430 nm. It was examined how the fluorescence of the G-clamp linker introduced to the 5' terminus of the oligonucleotide was changed by the overhang sequence during the formation of a double-strand with the target oligo-DNA or oligo-RNA. Accordingly, fluorescence spectra of oligonucleotide (ODN 1X) with a single G-clamp linker (PO) introduced to the 5' terminus and oligonucleotide (ODN 2X) with three G-clamp linkers (PO) introduced to the 5' terminus were measured in the form of a single-strand and in the form of a double-strand formed with the target oligo-DNAs or oligo-RNAs having the overhang sequences shown in Table 7. The results are shown in FIGS. 1 to 4.

As shown in FIG. 1, the fluorescence spectrum of the double-strand formed by the oligonucleotide (ODN 1X) with a single G-clamp linker (PO) introduced to the 5' terminus and the target oligo-DNA ("No YYYY" in FIG. 1) with no overhang was substantially the same as that of ODN 1X in the form of a single-strand. The fluorescence intensity decreased in the order of the double-strand ("CATA" in FIG. 1) formed by ODN 1X and the target oligo-DNA having the overhang with no guanine and the double-strands ("CGGT", "CTGG", "CAGT", and "CATG" in FIG. 1) formed by ODN 1X and the target oligo-DNAs having the overhang with a guanine.

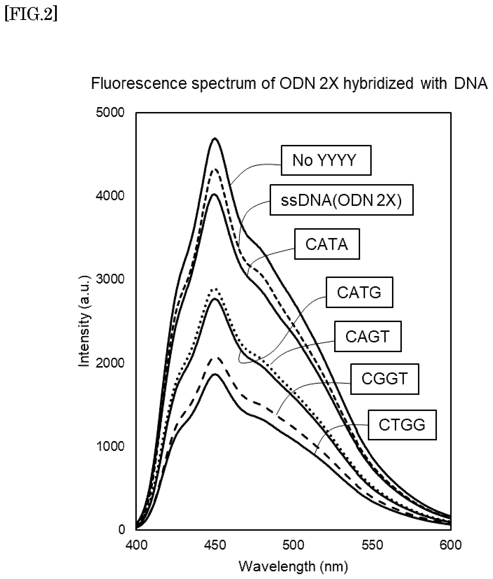

As shown in FIG. 2, the fluorescence spectrum of the double-strand ("No YYYY" in FIG. 2) formed by the oligonucleotide (ODN 2X) with three G-clamp linkers (PO) introduced to the 5' terminus and the target oligo-DNA with no overhang and the fluorescence spectrum of the double-strand ("CATA" in FIG. 2) formed by ODN 2X and the target oligo-DNA having the overhang with no guanine were substantially the same as that of ODN 2X in the form of a single-strand. The fluorescence intensity decreased in the order of the group consisting of the double-strands formed by ODN 2X and the oligo-DNAs having the overhang with one guanine ("CAGT" and "CATG" in FIG. 2) and the group consisting of the double-strands formed by ODN 2X and the oligo-DNAs having the overhang with two guanines ("CGGT" and "CTGG" in FIG. 2).

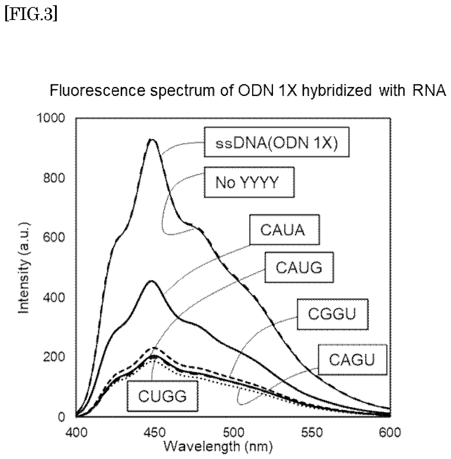

As shown in FIG. 3, the fluorescence spectrum of the double-strand ("No YYYY" in FIG. 3) formed by the oligonucleotide (ODN 1X) with a single G-clamp linker (PO) introduced to the 5' terminus and the target oligo-RNA with no overhang was substantially the same as that of ODN 1X in the form of a single-strand. The fluorescence intensity of the double-strand ("CAUA" in FIG. 3) formed by ODN 1X and the complementary RNA having the overhang with no guanine decreased to about half of that of ODN 1X. The fluorescence intensities of the other double-strands formed by ODN 1X and the oligo-DNAs having the overhang were substantially the same and the lowest.

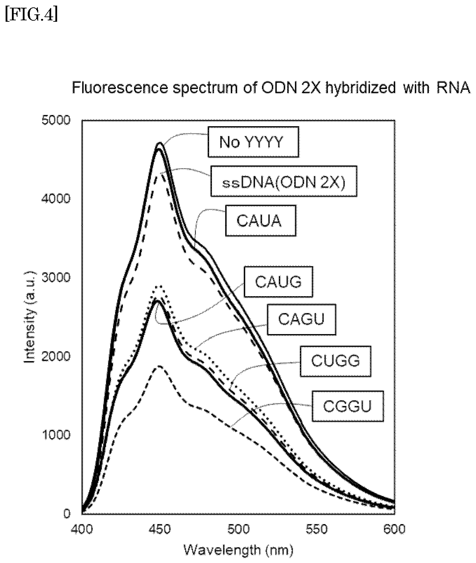

As shown in FIG. 4, the fluorescence spectrum of the double-strand ("No YYYY" in FIG. 4) formed by the oligonucleotide (ODN 2X) with three G-clamp linkers (PO) introduced to the 5' terminus and the oligo-RNA with no overhang and the fluorescence spectrum of the double-strand ("CAUA" in FIG. 4) formed by ODN 2X and the ohgo-RNA having the overhang with no guanine were substantially the same as that of ODN 2X in the form of a single-strand. The fluorescence intensity of the double-strand ("CGGU" in FIG. 4) formed by ODN 2X and the oligo-RNA having the overhang with two guanines at the center was the lowest.

### (3) Evaluation of Double-Strand Forming Ability and Base Discrimination Ability

A comparison reveals that the G-clamp linker (PO) obtained above and a dG-clamp (derived from a known compound) as shown below (Obika et al., Chem. Eur. J., 2021, 27, 2427-2438) are different from each other in that a sugar skeleton is present or absent, but have artificial bases that include a common nitrogen-containing heterocyclic structure (also referred to as a "G-clamp"), and it is also conceivable that the G-clamp linker (PO) also has a cytosine-selective base discrimination ability similarly to the dG-clamp. Accordingly, the melting temperatures of these compounds were measured and compared.

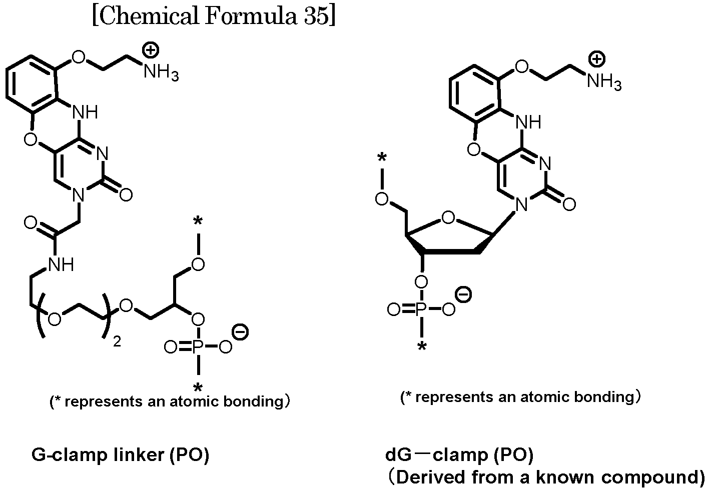

Specifically, oligonucleotides having the following sequences were synthesized and purified:
5'-d(GCGTTXTTTGCT)-3'
(1) X = G-clamp linker (PO) (ODN 3X)
(2) X = dG-clamp (PO)
(3) X = 2'-deoxycytidine (dC) (SEQ ID NO: 16)

The double-strand forming ability (binding affinity) of the oligonucleotides were examined using, as the target strands, a single-stranded oligo- DNAs 3'-d(CGCAAYAAACGA)-5' (SEQ ID NOs: 17 and 18) in which Y was a guanine (G) (SEQ ID NO: 17) and a single-stranded oligo-RNA 3'-r(CGCAAYAAACGA)-5' in which Y was a guanine (G) (SEQ ID NO: 18).

The double-strand forming ability of the oligonucleotides was examined by subjecting the different types of oligonucleotides and the target strands to an annealing treatment to form double strands, and then measuring their Tₘ values. More specifically, a sample solution of each oligonucleotide (final concentration: 2 µM) in a phosphate buffer (10 mM, pH 7.0) containing sodium chloride (final concentration: 100 mM) was heated at 100°C and then slowly cooled to room temperature. After that, the sample solution was cooled to 5°C under nitrogen stream before starting the measurement. The temperature was raised to 90°C at a rate of 0.5°C/minute while absorbance at 260 nm was plotted at intervals of 0.5°C. The Tₘ value was calculated using a median method, and a mean value of three independent measurements was adopted. The results are shown in Table 8.

**Table 8**

| X | *T*ₘ (°C) (*ΔT*ₘ: (G-clamp linker (PO) moiety or dG-clamp moiety)-dC) | |
|---|---|---|
| | Y= G | |
| | DNA | RNA |
| G-clamp linker (P O) | 30 (-15) | 34 (-20) |
| dG-clamp (PO) | 61 (+16) | 67 (+13) |
| dC | 45 | 54 |

Single-stranded oligo-DNAs 3'-d(CGCAAYAAACGA)-5' (SEQ ID NOs: 19 to 21) in which Y was a mismatch (i.e.,
(1) Y = cytosine (C) (SEQ ID NO: 19)
(2) Y = adenine (A) (SEQ ID NO: 20), and
(3) Y = thymine (T) (SEQ ID NO: 21)
were used as the target strands, and the double-strand forming ability of the oligonucleotides was examined in the same manner as in the operations performed for Table 8 above. The results are shown in Table 9.

**Table 9**

| X | *Tₘ* (°C) (*ΔT*ₘ: Mismatch-Match) | | | |
|---|---|---|---|---|
| | Macch | Mismatch | | |
| | Y=G | Y=C | Y=A | Y=T |
| G-clamp linker (PO) | 30 | 17 (-13) | 13 (-17) | 16 (-14) |
| d G-clamp (PO) | 61 | 24 (-37) | 38 (-23) | 34 (-27) |
| dC | 45 | 24 (-21) | 32 (-13) | 29 (-16) |

Furthermore, single-stranded oligo-RNAs 3'-r(CGCAAYAAACGA)-5' (SEQ ID NOs: 22 to 24) in which Y was a mismatch (i.e.,
(1) Y = cytosine (C) (SEQ ID NO: 22)
(2) Y = adenine (A) (SEQ ID NO: 23), and
(3) Y = uracil (U) (SEQ ID NO: 24))
were used as the target strands, and the double-strand forming ability of the oligonucleotides was examined in the same manner as in the operations performed for Table 7 above. The results are shown in Table 10.

**Table 10**

| X | *T*ₘ (°C) (*ΔT*ₘ: Mismatch - Match) | | | |
|---|---|---|---|---|
| | Match | Mismatch | | |
| | Y=G | Y=C | Y=A | Y=U |
| G-clamp linker (PO) | 34 | 13 (-21) | 15 (-19) | 23 (-11) |
| d G-clamp (PO) | 67 | 38 (-29) | 44 (-23) | 41 (-26) |
| dC | 54 | 32 (-22) | 39 (-15) | 31 (-23) |

As shown in Tables 8 to 10, when the oligonucleotide (ODN 3X) with the G-clamp linker (PO) introduced was used, the Tₘ values of the target single-stranded oligo- DNA and the single-stranded oligo-RNA in which the base to be paired was a guanine serving as a matching base significantly decreased relative to the cases of using the corresponding oligonucleotides with the dG-clamp (PO) and dC. Meanwhile, when the oligonucleotide (ODN 3X) with the G-clamp linker (PO) introduced was used, the Tₘ values of the target single-stranded oligo-DNAs and the single-stranded oligo-RNAs in which the base to be paired was a cytosine, adenine, or thymine were lower by 10°C or more relative to the case of using target single-stranded oligo-DNA and the single-stranded oligo-RNA in which the base to be paired was a guanine. These results show that the oligonucleotide (ODN 3X) with the G-clamp linker (PO) introduced has lower affinity for a matching guanine sequence relative to the case of using the dG-clamp (PO) or dC, but has the base discrimination ability similarly to the dG-clamp (PO) and dC.

Example 4: Production and Evaluation of Antisense Oligonucleotide for Administration to Mouse

### (1) Procedure for Producing Antisense Oligonucleotide for Administration to Mouse

In order to test the expression suppressing effect of an antisense oligonucleotide (ASO) to which the G-clamp linker (PO) or G-clamp linker (PS) (these may also be collectively referred to as a "G-clamp linker") has been conjugated in the viscera of a mouse, ASOs to which the G-clamp linkers listed in Table 12 are conjugated were designed and synthesized. Gapmer-type PS oligonucleotide that targets the mouse MALAT1 (Metastasis Associated in Lung Adenocarcinoma Transcript-1) was produced by substituting cET in the sequence reported by G. Hung et al. (Nucleic Acid Then, 2013, 23, 369-378) with 2',4'-BNA/LNA. Four types of ASOs, GCL(1O), GCL(3O), GCL(1S), and GCL(3S), having a configuration in which one or three G-clamp linkers (PO) or G-clamp linkers (PS) are introduced to the 5' terminus of the ASO above, were designed. The conjugates were named according to the following definition: "GCL" before a parenthesis means an ASO to which the G-clamp linker

(PO) or G-clamp linker (PS) is conjugated, a figure in the parenthesis after "GCL" represents the number of the introduced G-clamp linkers (PO) or G-clamp linkers (PS), and "O" and "S" represent the types of bonds included in the G-clamp linker and are "PO" and "PS", respectively. Furthermore, for example, "GCL" without parentheses includes all of the four types of conjugates. In addition, for example, "GCL3" includes two types of conjugates, GCL(3O) and GCL(3S).

For comparison purposes, GCS (four types) and PXS (four types) having a configuration in which a dG-clamp or a dtC^{O} is conjugated to the ASO above in the same manner were also synthesized. (These structures are shown in Table 12).

GCL(1O), GCL(1S), GCL(3O), and GCL(3S) were synthesized using Compound 18 (amidite with the G-clamp linker moiety) with an automated DNA synthesizer (nS-8 Oligonucleotides Synthesizer manufactured by GeneDesign). Oligonucleotide synthesis was performed on a 1.0-pmol scale in the DMTr-on mode. 5-[3,5-bis(trifluoromethyl)phenyl]-1H-tetrazole (Activator 42 (registered trademark)) was used as an activator in all coupling processes. Aqueous iodine was used as an oxidizing agent, and 3-[(dimethylaminomethylene)amino]-3H-1,2,4-dithiazole-5-thione, DDTT, was used as a sulfurizing agent. Compound 18, natural forms, and the 2',4'-BNA/LNA altered phosphoramidites other than ^{m}C were prepared at a concentration of 100 mM in an ANC solution, and the ^{m}C 2',4'-BNA/LNA altered phosphoramidite was prepared at a concentration of 100 mM in an ACN/THF (1/1) solution. The coupling times for Compound 18, the natural forms, and the 2',4'-BNA/LNA altered phosphoramidites were 180 seconds, 30 seconds, and 12 minutes, respectively. The oligonucleotide supported by a solid (CPG) was cleaved, and the protecting groups for the nucleic acid bases and the phosphorodiester bond were removed at 65°C for 15 minutes using a mixed solution (an aqueous solution of methylamine (40%) and an aqueous solution of ammonia (28%) (volume ratio = 1:1)).

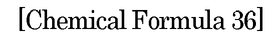

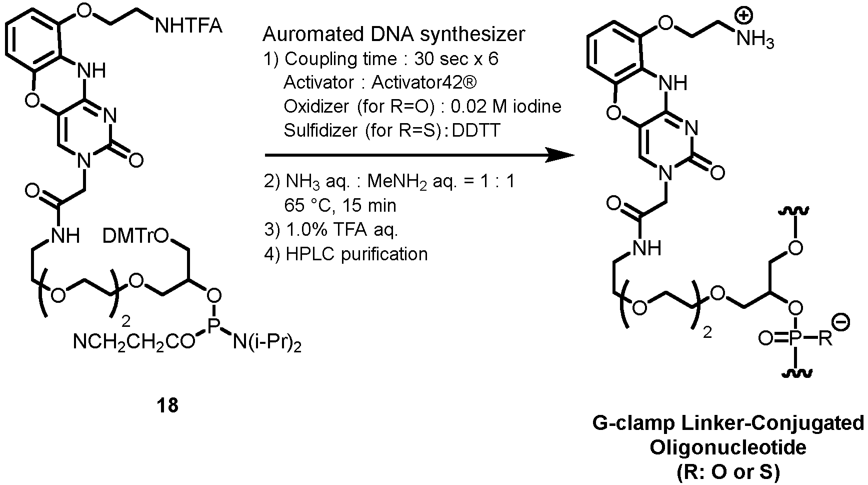

DMTr was removed from the crude oligonucleotide having a DMTr group, and the oligonucleotide was roughly purified using a simple reverse-phase column (Waters Sep-Pak (registered trademark) Plus C18 Environmental Cartridges). Specifically, the crude oligonucleotide was added to the Sep-Pak, and then the column was washed using a 10% aqueous solution of ACN. Subsequently, DMTr was removed using a 1% aqueous solution of trifluoroacetic acid, and the oligonucleotide was eluted using a 40% aqueous solution of MeOH. Next, the oligonucleotide was purified through HPLC with an XBridge^{™} OST C18 2.5 pm (10 × 50 mm) reverse-phase HPLC column manufactured by Waters using a gradient elution method in which 100 mM triethylammonium acetate (TEAA) buffer (pH=7.0) was used as an eluent A and MeOH was used as an eluent B.

The components of the purified oligonucleotide were examined through reverse-phase HPLC with XBridge^{™} OST C18 2.5 pm (4.6 × 50 mm) manufactured by Waters and mass spectroscopy with a MALDI-TOF mass spectrometer. The overall yield was calculated from the UV absorbance at 260 nm. Note that the measurement method and the conditions used for the reverse-phase HPLC were as shown in Table 11 below

GCS(1O), GCS(1S), GCS(3O), GCS(3S), PXS(1O), PXS(1S), PXS(3O) and PXS(3S) were synthesized and purified by GeneDesign. The sequence information of the antisense oligonucleotides is as shown in Table 12.

**Table 11**

| General Conditions |
|---|
| Preparative chromatography: Waters XBridge^{™} OST C18 2.5µm (10×50mm) |
| Preparative chromatography: Waters XBridge^{™} OST C18 2.5µm (4.6×50 mm) |
| Flow rate of preparative conditions: 4.0mL/min |
| Flow rate of preparative conditions: 1.0mL/min |
| Wavelength of UV for oligonucleotide detection: 260nm |
| Column oven temperature: 50°C |
| Eluent A: 100 mM TEAA (pH 7.0) buffer |
| Eluent B: MeOH |
| MeOH gradient in both preparative and analytical conditions (20 min) |
| Retention time (Rt) (min) (Analytical column conditions); |
| 5-70%; ODN GCL(1O) (Rt: 11.2mm). ODN GCL(3O) (Rt: 11.5min), ODN GCL (IS) (Rt: 11.3min), ODN GCL(3S) (Rt : 11.8min) |

**Table 12**

| ASO | Sequence (5'-3') | | MALDI-TOF-Mass | |
|---|---|---|---|---|
| | | | Calculated [M-H]⁻ | Found [M-H]⁻ |
| GCL(1O) | 5'-XC^T^A^g^t^t^c^a^c^t^g^a^a^T^G^C-3' | (SEQ ID NO. 25) | 5877.8 | 5879.2 |
| GCI.(30) | 5'-XXXC^T^A^g^t^t^c^a^c^t^g^a^a^T^G^C-3' | (SEQ ID NO. 26) | 7048.8 | 7050.4 |
| GCL(1S) | 5'-X^C^T^A^g^t^t^c^a^c^t^g^a^a^T^G^C-3' | (SEQ ID NO. 27) | 5893.8 | 5893.9 |
| GCL(3S) | 5'-X^X^X^C^T^A^g^t^t^c^a^c^t^g^a^a^T^G^C-3' | (SEQ ID NO. 28) | 7096.9 | 7098.1 |
| GCS(1O) | 5'-YC^T^A^g^t^t^c^a^c^t^g^a^a^T^G^C-3' | (SEQ ID NO. 29) | 5732.6 | 5731.7 |
| GCS(3O) | 5'-YYYC^T^A^g^t^t^c^a^c^t^g^a^a^T^G^C-3' | (SEQ ID NO. 30) | 6610.9 | 6608.3 |
| GCS(1S) | 5'-Y^C^T^A^g^t^t^c^a^c^t^g^a^a^T^G^C-3' | (SEQ ID NO. 31) | 5748.5 | 5747.7 |
| GCS(3S) | 5'-Y^Y^Y^C^T^A^g^t^t^c^a^c^t^g^a^a^T^G^C-3' | (SEQ ID NO. 32) | 6654.5 | 6656.5 |
| PXS(1O) | 5'-ZC^T^A^g^t^t^c^a^c^t^g^a^a^T^G^C-3' | (SEQ ID NO. 33) | 5671.9 | 5672.6 |
| PXS(3O) | 5'-ZZZC^T^A^g^t^t^c^a^c^t^g^a^a^T^G^C-3' | (SEQ ID NO. 34) | 6433.1 | 6431.1 |
| PXS(1S) | 5'-Z^C^T^A^g^t^t^c^a^c^t^g^a^a^T^G^C-3' | (SEQ ID NO. 35) | 5688.2 | 5688.7 |
| PXS(3S) | 5'-Z^Z^Z^C^T^A^g^t^t^c^a^c^t^g^a^a^T^G^C-3' | (SEQ ID NO. 36) | 6477.0 | 6479.3 |

| | | | | |
|---|---|---|---|---|
| X:G-claim linker moiety Y:dG-clamp moiety Z:dtC° moiety ^:phosphorothioate bonding (PS bonding) a, g, c, t: dA, dG, dC, T A, G, C, T : 2',4'-BNA/LNA with adenine, guanine, 5-methylcytosine and thymine | | | | |

The terms "dG-clamp moiety", "dG-clamp (PO)", "dG-clamp linker (PS)", "dtC^{O} moiety", "dtC^{O} (PO)", and "dtC^{O} (PS)" as used herein respectively refer to the following structures.

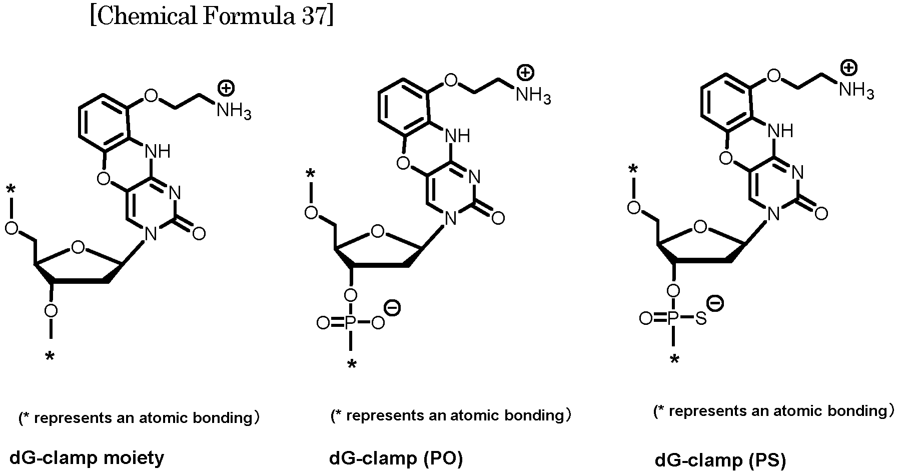

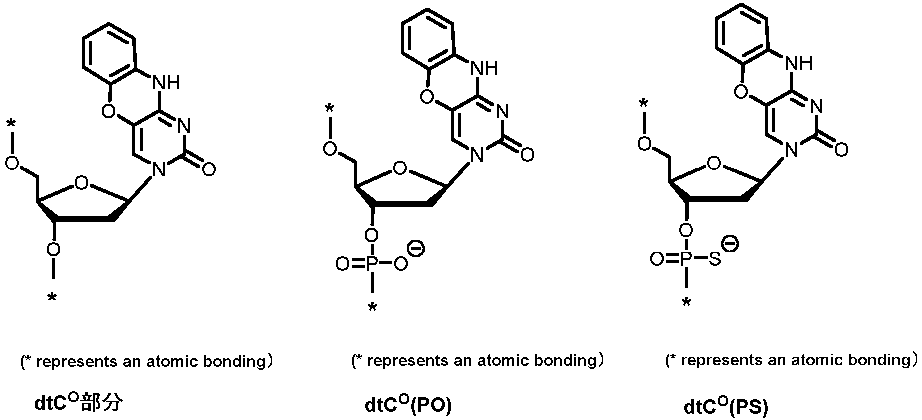

### (2) Evaluation of Tₘ Value of Antisense Oligonucleotide for Administration to Mouse

Influences of the G-clamp linker, the dG-clamp, and the dtC^{O} introduced to the 5' termini of GCL, GCS, and PXS on binding to an mMALAT1-RNA target sequence were evaluated through Tₘ measurement using a model sequence of the mMALAT1-RNA. The target model RNA sequence was a 20-mer sequence constituted by a sequence (16 mer) complementary to the ASO and GAAG serving as an overhang region linked to the 3' terminus of the 16-mer sequence. In the same manner as described above, the double-strand forming ability of the antisense oligonucleotides for administration to a mouse was examined by subjecting the different types of oligonucleotides and the target strands to an annealing treatment to form double strands, and then measuring their Tₘ values. More specifically, a sample solution of each oligonucleotide (final concentration: 2 µM) in a phosphate buffer (10 mM, pH 7.0) containing sodium chloride (final concentration: 100 mM) was heated at 100°C and then slowly cooled to room temperature. After that, the sample solution was cooled to 5°C under nitrogen stream before starting the measurement. The temperature was raised to 90°C at a rate of 0.5°C/minute while absorbance at 260 nm was plotted at intervals of 0.5°C. The Tₘ value was calculated using a median method, and a mean value of three independent measurements was adopted. The results are shown in Table 13.

**Table 13**

| ASO | *T*ₘ (*ΔT*ₘ = *T*ₘ - *T*ₘ (naked ASO/oRNA)) (°C) | | | |
|---|---|---|---|---|
| | x:1 | | x:3 | |
| | y: O | y: S | y: O | y: S |
| GCL(xy) | 58 (+5) | 57 (+4) | 59 (+6) | 58 (+5) |
| PXS(xy) | 58 (+5) | 58 (+5) | 59 (+6) | 58 (+5) |
| GCS(xy) | 67 (+14) | 66 (+13) | 71 (+18) | 71 (+18) |

| | | | | |
|---|---|---|---|---|
| Target RNA sequence (oRNA):3'-r(GAAGGAUCAAGUGACUUACG)-5' (The 4-mer on the 3' si de was the overhang region.)(SEQ ID NO. 37) Tm value of the naked ASO/oRNA was 53°C. | | | | |

Regarding GCL and PXS, the Tₘ value remained substantially the same irrespective of the number of the ligands. Also, regarding GCL, PXS, and GCS, the Tₘ value remained substantially the same irrespective of whether the PO bond or the PS bond was included, and the Tₘ values of GCL and PXS were higher than that of naked ASO by 4°C to 6°C while the Tₘ value of GCS was higher than that of naked ASO by even 13°C to 18°C. It is conceivable that, since the 5'-terminal base of the overhang (3'-GAAG-5') in the target oRNA was a guanine, the G-clamp linker, the dtC^{O}, and the dG-clamp in GCL, PXS, and GCS formed a hydrogen bond with the complementary matching guanine of the oRNA, which contributed to stabilization.

### (3) Evaluation of Antisense Oligonucleotide for Administration to Mouse Administration of ASO and Collection of Viscera

Mice (Balb/c, female, 5 weeks old, seven mice) were purchased from Japan SLC, Inc. and delivered to an animal experimentation facility. After the mice were acclimated for at least 5 to 7 days, a physiological saline solution of GCL, GCS, or PXS was prepared and once administered to the caudal vein such that a dose per mouse was 100 pg. As a comparison, a physiological saline solution of an unconjugated ASO (ASO to which a ligand and a linker are not linked: "naked ASO") was prepared and was administered to the caudal vein such that a dose per mouse was 100 pg. A physiological saline solution was used as a negative control.

The mice were each anesthetized through intraperitoneal administration of a triple anesthetic combination (medetomidine, midazolam, butorphanol) 72 hours after the administration of the ASO, and were sacrificed by exsanguination through laparotomy followed by incision of the abdominal aorta. Then, 25 to 50 mg of the skeletal muscle tissue was collected from the thigh and was placed in a 2-mL tube in 96-well Collection Microtubes (Qiagen) in which 500 pL of RNAprotect Tissue Reagent (Thermo) had been dispensed per tube in advance, and then the tissue fragment was stored (-30°C) while being kept immersed in the reagent.

### Preparation of Trial RNA, Reverse Transcription, and Real-Time PCR

MagMAX mirVana Total RNA Isolation Kit (Thermo; referred to as "mirVana kit" hereinafter) was used to extract total RNA from the collected tissue fragment. The 96-well Collection Microtubes in which the tissue fragment was stored were centrifuged (1000×g, 3 minutes, 4°C) and the RNAprotect Tissue Reagent was removed. Then, a lysis buffer (supplemented with 0.7% 2-mercaptoethanol) and one stainless-steel bead (5-mm diameter, QIAGEN), which are attached to the mirVana kit, were added to each tube of the Collection Microtubes, and a tissue homogenate was produced by repeating homogenization treatment at 30 Hz for 2 minutes at room temperature 5 or more times using TissueLyser II (QIAGEN).

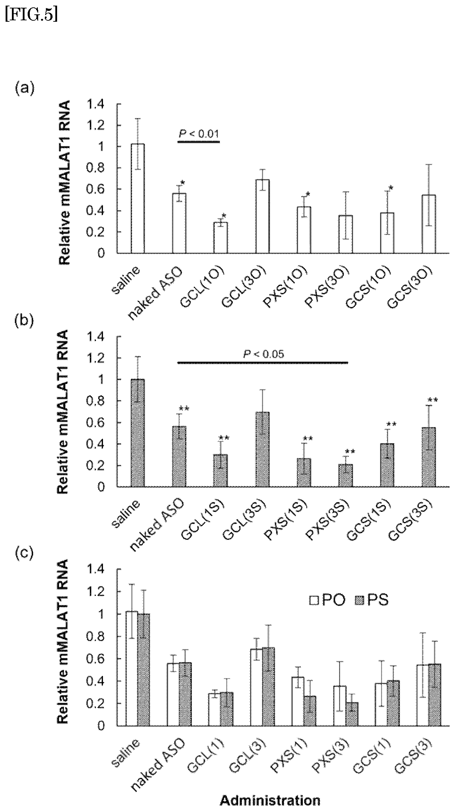

The extraction of total RNA from the tissue homogenate and the purification thereof were performed in accordance with the protocol of the mirVana kit, and the processes were automated using KingFisher Flex (Thermo). The concentrations of RNA samples were determined using Quant-iT RiboGreen RNA Assay Kit (Thermo). A reverse transcription product was prepared from about 10 ng of the total RNA using High-Capacity cDNA Reverse Transcription Kit (Thermo). The reaction was conducted in accordance with the protocol of the kit. Each reverse transcription product was subjected to real-time PCR conducted using PowerUp SYBR Green Master Mix (Thermo). Gapdh was used as the internal control of the PCR. The PCR reaction was conducted using StepOnePlus Real-Time PCR System (Thermo), and in the reacton, heat treatment was performed at 95°C for 20 seconds, followed by 45 cycles of thermal denaturation at 95°C for 3 seconds and extension reaction at 60°C for 30 seconds. The expression level of Malat1 was analyzed by comparing Ct values obtained from amplification curves of Malat1 and Gapdh (ΔΔCt method). The results are shown in FIG. 5.

The sequences of PCR primes used in the test above are shown below:
mMalat1_F4: 5'-d(ACATTCCTTGAGGTCGGCAA)-3' (SEQ ID NO:38)
mMalat1_R4: 5'-d(CACCCGCAAAGGCCTACATA)-3' (SEQ ID NO: 39)
mGapdh_F3: 5'-d(TCACCACCATGGAGAAGGC)-3' (SEQ ID NO:40)
mGapdh_R3:5'-d(GCTAAGCAGTTGGTGGTGCA)-3' (SEQ ID NO:41)

As shown in FIG. 5, GCL(1O) in which a single G-clamp linker had been introduced to the ASO via PO bonds statistically significantly suppressed the expression level of the mMALAT1 RNA in the skeletal muscle relative to the case of using the naked ASO, indicating that GCL(1O) had very high activity against at least the skeletal muscle among viscera, organs, and tissues other than the liver. This result shows that the G-clamp linker produced in Example 1 is applicable to a drug delivery system formulation for selectively delivering a nucleic acid drug to the skeletal muscle.

When PXS(3S) in which three dtC^{O}s had been introduced to the ASO via PS bonds was administered, the expression level of the mMALTA1 RNA in the skeletal muscle was statistically significantly suppressed relative to the case of using the naked ASO, indicating that PXS(3S) had very high activity in the skeletal muscle. This result shows that the dtC^{O} is also applicable to a drug delivery system formulation for delivering a nucleic acid drug to at least the skeletal muscle among viscera, organs, and tissues other than the liver.

### Industrial Applicability

The present invention is useful in, for example, the development and production of pharmaceuticals.

## Claims

1. A compound represented by Formula (I) below or a pharmaceutically acceptable salt thereof 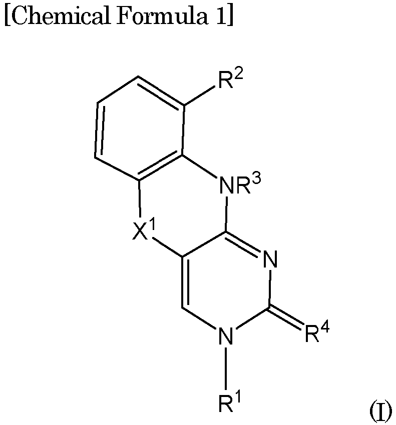 (in Formula (I),
R¹ is a group represented by Formula (II) below: 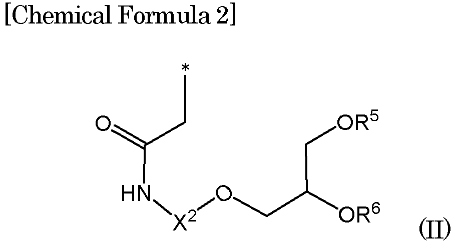 (in Formula (II),
X² is a group represented by the formula below: 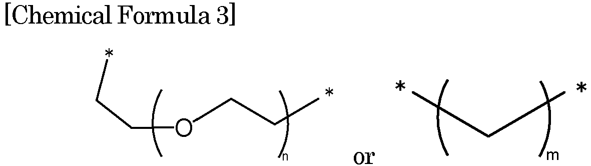 (where n is any of integers of 2 to 4,
m is any of integers of 2 to 4, and
* is an atomic bonding),
R⁵ is a hydrogen atom, an alkyl group having 1 to 7 carbon atoms that may be branched or cyclic, or a hydroxy group protecting group for nucleic acid synthesis,
R⁶ represents a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 10 carbon atoms that may have any one or more substituents selected from an a group and may include a heteroatom, an aralkyl group with an aryl moiety having 3 to 12 carbon atoms that may have any one or more substituents selected from the a group and may include a heteroatom, an acyl group that may have any one or more substituents selected from the a group, a silyl group that may have any one or more substituents selected from the a group, a phosphate group that may have any one or more substituents selected from the a group, a phosphate group protected by a protecting group for nucleic acid synthesis, or -P(R¹⁴)R¹⁵ (where R¹⁴ and R¹⁵ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has alkyl groups having 1 to 6 carbon atoms),
the a group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms, and
* is an atomic bonding),
R² is a hydrogen atom, a hydroxy group, a nitro group, an amino group, or a group represented by Formula (III) below: 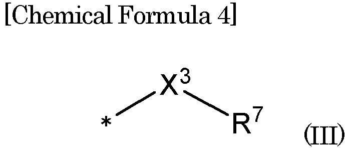 (in Formula (III),
X³ is an oxygen atom or a sulfur atom, and
R⁷ is a group represented by the formula below: 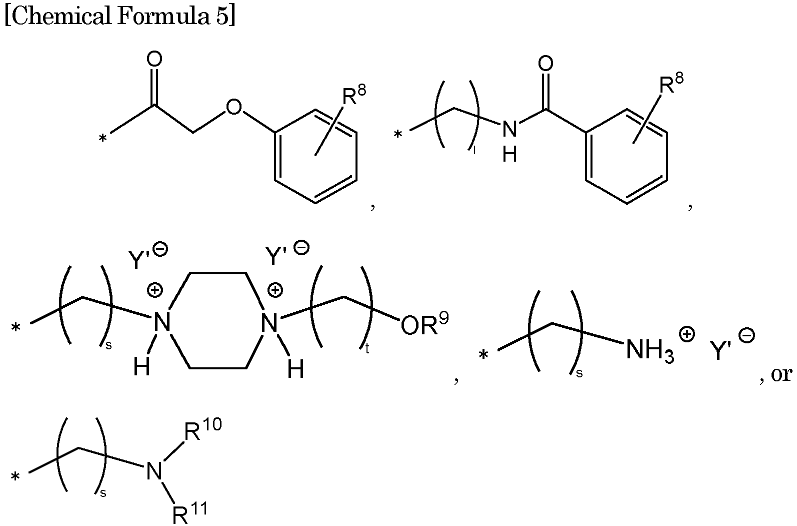 (where
R⁸ is a hydrogen atom, a hydroxy group, an alkyl group having 1 to 7 carbon atoms that may be branched or cyclic, or an alkoxy group having 1 to 7 carbon atoms that may be branched or cyclic,
R⁹ is a hydrogen atom, or an alkyl group having 1 to 7 carbon atoms that may be branched or cyclic,
R¹⁰ and R¹¹ are each independently a hydrogen atom, an alkyl group having 1 to 7 carbon atoms that may be branched or cyclic, or an amino group protecting group,
Y' is a pharmaceutically acceptable anion,
s is any of integers of 2 to 4,
t is any of integers of 2 to 4,
1 is any of integers of 1 to 5, and
* is an atomic bonding)),
R³ is a hydrogen atom, an alkyl group having 1 to 7 carbon atoms that may be branched or cyclic, or an amino group protecting group,
R⁴ is an oxygen atom or a sulfur atom, and
X¹ is an oxygen atom or a sulfur atom).

2. The compound or pharmaceutically acceptable salt thereof according to claim 1, wherein Formula (I) is represented by Formula (Ia) below: 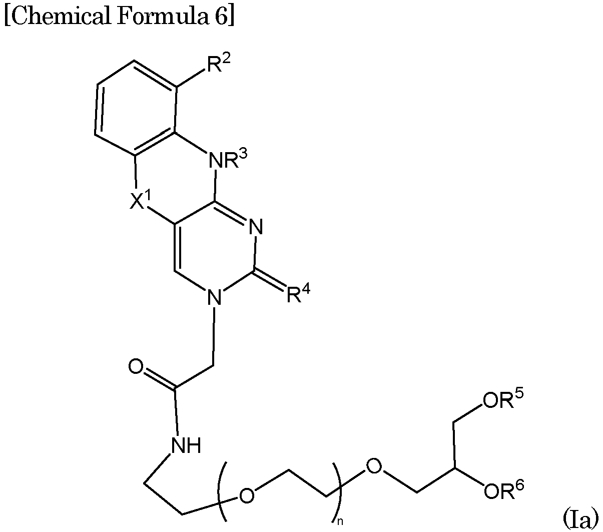 (in Formula (Ia), R², R³, R⁴, R⁵, R⁶, X¹, and n are as defined above).

3. The compound or pharmaceutically acceptable salt thereof according to claim 1, wherein Formula (I) is represented by Formula (Ib) below: 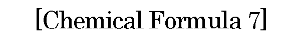 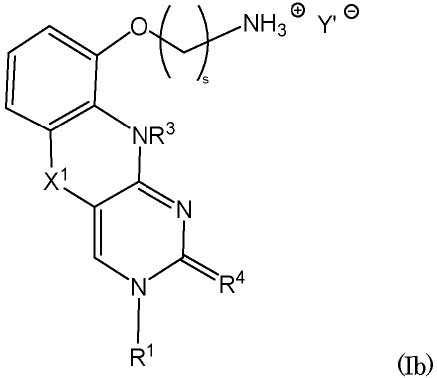 (in Formula (Ib), R¹, R³, R⁴, X¹, Y', and s are as defined above).

4. The compound or pharmaceutically acceptable salt thereof according to claim 1, wherein Formula (I) is represented by Formula (Ic) below: 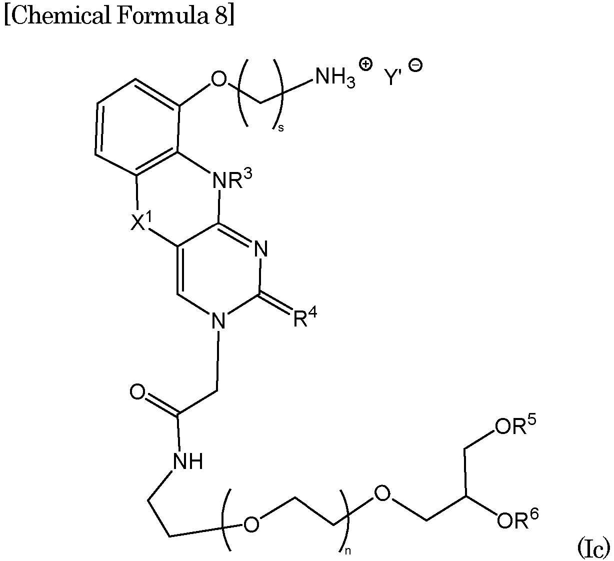 (in Formula (Ic), R³, R⁴, R⁵, R⁶, X¹, Y', n, and s are as defined above).

5. A drug delivery system formulation comprising a ligand derived from the compound or pharmaceutically acceptable salt thereof according to any one of claims 1 to 4.

6. A drug delivery system formulation comprising
a ligand derived from a compound represented by Formula (I') below or a pharmaceutically acceptable salt thereof 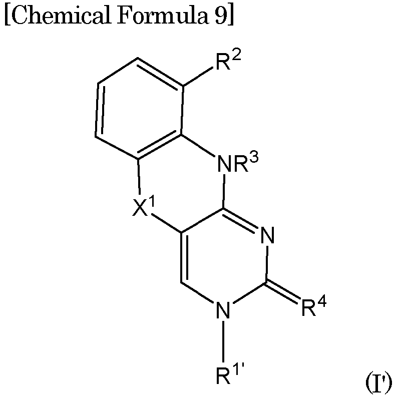 (in Formula (I'),
R^{1'} is
a hydrogen atom, an alkyl group having 1 to 7 carbon atoms that may be branched or cyclic, an alkoxy group having 1 to 7 carbon atoms that may be branched or cyclic, or an alkylthio group having 1 to 6 carbon atoms that may be branched; or
a pentose moiety that includes a chemical structure selected from the group consisting of a ribos- 1-yl structure and a 2-deoxyribos- 1-yl structure,
R² is a hydrogen atom, a hydroxy group, a nitro group, an amino group, or a group represented by Formula (III) below: 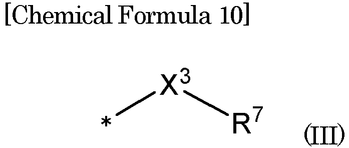 (in Formula (III),
X³ is an oxygen atom or a sulfur atom, and
R⁷ is a group represented by the formula below: 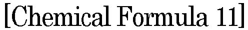 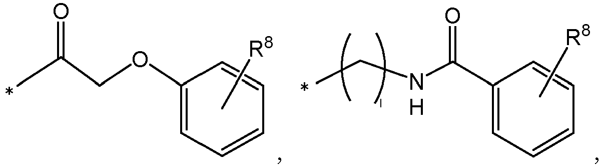 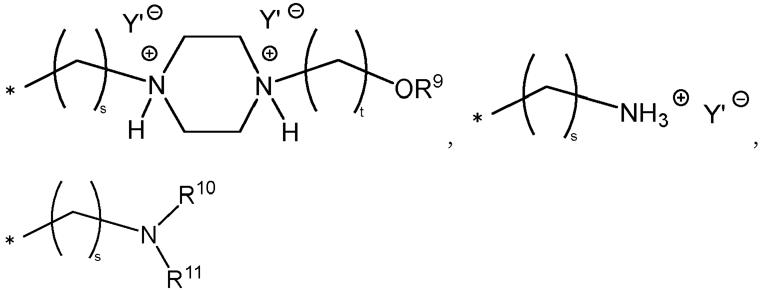 or (where
R⁸ is a hydrogen atom, a hydroxy group, an alkyl group having 1 to 7 carbon atoms that may be branched or cyclic, or an alkoxy group having 1 to 7 carbon atoms that may be branched or cyclic,
R⁹ is a hydrogen atom, or an alkyl group having 1 to 7 carbon atoms that may be branched or cyclic,
R¹⁰ and R¹¹ are each independently a hydrogen atom, an alkyl group having 1 to 7 carbon atoms that may be branched or cyclic, or an amino group protecting group,
Y' is a pharmaceutically acceptable anion,
s is any of integers of 2 to 4,
t is any of integers of 2 to 4,
1 is any of integers of 1 to 5, and
* is an atomic bonding)),
R³ is a hydrogen atom, an alkyl group having 1 to 7 carbon atoms that may be branched or cyclic, or an amino group protecting group,
R⁴ is an oxygen atom or a sulfur atom, and
X¹ is an oxygen atom or a sulfur atom).

7. The drug delivery system formulation according to claim 6, wherein R^{1'} in Formula (I') is the pentose moiety.

8. The drug delivery system formulation according to claim 7, wherein the pentose moiety is a moiety obtained through desorption of a base moiety from an artificial nucleic acid monomer.

9. The drug delivery system formulation according to claim 8, wherein the artificial nucleic acid monomer is a 2',4'-BNA/LNA monomer.

10. The drug delivery system formulation according to any one of claims 5 to 9, wherein the ligand is contained in the form of a conjugate where the ligand is linked to a polynucleic acid.

11. The drug delivery system formulation according to claim 10, wherein the ligand is linked to the 5' terminus of the polynucleic acid.

12. The drug delivery system formulation according to claim 10 or 11, wherein, in the conjugate, the number of the ligands linked to the polynucleic acid is one to three per polynucleic acid molecule.

13. The drug delivery system formulation according to any one of claims 10 to 13, wherein the polynucleic acid is an oligonucleotide.

14. The drug delivery system formulation according to claim 13, wherein the oligonucleotide is an antisense oligonucleotide, siRNA, or nucleic acid aptamer.

15. The drug delivery system formulation according to any one of claims 5 to 14, which is to be used for delivery to a skeletal muscle.

16. A pharmaceutical comprising the drug delivery system formulation according to any one of claims 5 to 15.
